(19) Europäisches Patentamt
European Patent Office
Office européen des brevets

(11) Veröffentlichungsnummer: **0 185 909**
**B1**

(12) # EUROPÄISCHE PATENTSCHRIFT

(45) Veröffentlichungstag der Patentschrift: **21.02.90**

(51) Int. Cl.[5]: **C 07 D 231/22**, C 07 D 401/12, C 07 D 498/04, A 61 K 31/495 // (C07D498/04, 265:00, 231:00)

(21) Anmeldenummer: **85114466.7**

(22) Anmeldetag: **14.11.85**

(54) **5-Alkyl-1-phenyl-2-piperazinoalkylpyrazolin-3-on-Verbindungen sowie Verfahren und Zwischenprodukte zu ihrer Herstellung und diese Verbindungen enthaltende Arzneimittel.**

(30) Priorität: **24.11.84 DE 3442860**

(43) Veröffentlichungstag der Anmeldung: **02.07.86 Patentblatt 86/27**

(45) Bekanntmachung des Hinweises auf die Patenterteilung: **21.02.90 Patentblatt 90/8**

(84) Benannte Vertragsstaaten: **AT BE CH DE FR GB IT LI LU NL SE**

(56) Entgegenhaltungen:
**EP-A- 0 072 960**
**GB-A- 1 473 000**
**US-A- 3 362 956**

**CHEMICAL ABSTRACTS, Band 98, Nr. 9, 28. Februar 1983, Seite 625, Spalte 1, Zusammenfassungsnr. 72018h, Columbus, Ohio, US; T. UEDA et al.: "Synthesis of pyrazolone derivatives. XLII. Synthesis and analgesic activity of 2-methyl-1-phenyl-6,7-dihydro-1H, 5H-pyrazolo[5,1-b][1,3]oxazin-8-ium bromide"**
**HOUBEN-WEYL "Methoden der Organischen Chemie", Band XI/1, Stickstoffverbindungen II, 4. Auflage, Seiten 108-111, Georg Thieme Verlag, Stuttgart; H. GLASER et al.: "Amine 1. Herstellung"**
**C.J. ESTLER (Hrsg.) Lehrbuch der allgemeinen und**

(73) Patentinhaber: **Kali-Chemie Pharma GmbH, Hans-Böckler-Allee 20 Postfach 220, D-3000 Hannover 1 (DE)**

(72) Erfinder: **Jasserand, Daniel, 2 Allée d'Andrezieux, F-75018 Paris (FR)**
Erfinder: **Christen, Marie-Odile, 240 Rue du Faubourg St. Honoré, F-75008 Paris (FR)**
Erfinder: **Biard, Dominique, 21 La Butte Eglantine, F-95160 Eragny (FR)**
Erfinder: **Yavordios, Dimitri, 536 Avenue Dubanchet, F-01400 Chatillon s/Chalaronne (FR)**

(74) Vertreter: **Lauer, Dieter, Dr., c/o Kali-Chemie AG Postfach 220 Hans-Böckler-Allee 20, D-3000 Hannover 1 (DE)**

(56) Entgegenhaltungen: (Fortsetzung)
**systematischen Pharmakologie und Toxikologie, Verlag F.K. Schattauer 1983, S.74,77,78,81,83.**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue 5-Alkyl-1--phenyl-2-piperazinoalkylpyrazolin-3-on-Verbindungen und deren Salze sowie diese Verbindungen enthaltende pharmazeutische Zubereitungen und Verfahren und Zwischenprodukte zur Herstellung dieser Verbindungen.

Aus der deutschen Offenlegungsschrift 3 132 915 sind 2-Piperazinoalkyl-1,5-diphenylpyrazolin-3-on--Verbindungen mit antiallergischen Eigenschaften bekannt.

Der vorliegenden Erfindung liegt die Aufgabe zugrunde, neue 2-Piperazinoalkyl-1-phenylpyrazolin--3-on-Verbindungen mit wertvollen pharmakologischen Eigenschaften zu entwickeln.

Es wurde nun gefunden, daß die neuen 5-Alkyl-1--phenyl-2-piperazinoalkylpyrazolin-3-on-Verbindungen wertvolle pharmakologische Eigenschaften, insbesondere ausgeprägte antiallergische Eigenschaften bei guter therapeutischer Breite und geringer Toxizität besitzen und sich durch ein verbessertes Wirkungsprofil auszeichnen. So weisen die erfindungsgemäßen Verbindungen neben ausgeprägten antiallergischen Eigenschaften auch periphere Antihistamin- und Antiserotonin-Wirkungen sowie ödemhemmende Eigenschaften auf.

Aufgrund dieser Eigenschaften sind die neuen Verbindungen als Arzneimittel zur Behandlung von allergischen Erkrankungen wie beispielsweise allergisch bedingtem Asthma, Heuschnupfen oder allergisch bedingten Entzündungen, geeignet.

Die vorliegende Erfindung betrifft daher neue 5-Alkyl-1-phenyl-2-piperazinoalkylpyrazolin-3-on-Verbindungen der allgemeinen Formel I

(siehe Formel I)

worin

$R_1$ eine geradkettige, verzweigte oder cyclische Alkylgruppe mit bis zu 6 Kohlenstoffatomen bedeutet,

$R_2$ Wasserstoff, Halogen, Nitro, Cyano, Hydroxy, Alkyl mit 1-4 Kohlenstoffatomen, Alkoxy mit 1-4 Kohlenstoffatomen, oder eine $R_5$-CO-Gruppe, worin $R_5$ für Alkoxy mit 1-4 Kohlenstoffatomen, Alkyl mit 1-4 Kohlenstoffatomen oder Hydroxy steht, bedeutet, und

$R_3$ Wasserstoff, Halogen, Alkyl mit 1-4 Kohlenstoffatomen oder Alkoxy mit 1-4 Kohlenstoffatomen bedeutet,

Z eine Alkylenkette mit 2-4 Kohlenstoffatomen bedeutet,

$R_4$ eine Phenylgruppe der Formel a oder eine Pyridylgruppe der Formel b bedeutet,

(siehe Formeln a und b)

worin

$R_6$ Wasserstoff, Halogen, Alkyl mit 1-4 Kohlenstoffatomen oder Alkoxy mit 1-4 Kohlenstoffatomen bedeutet, und

$R_7$ Wasserstoff, Halogen, Alkyl mit 1-4 Kohlenstoffatomen oder Alkoxy mit 1-4 Kohlenstoffatomen bedeutet,

sowie deren Säureadditionssalze.

Sofern in den Verbindungen der Formel I die Substituenten $R_2$, $R_3$ und $R_4$ niedere Alkylgruppen enthaltende Substituenten darstellen, können diese gerade oder verzweigt sein und 1-4, vorzugsweise 1-2 Kohlenstoffatome enthalten.

Sofern die Substituenten $R_2$, $R_3$ oder $R_4$ Halogen darstellen oder enthalten, kommen insbesondere Fluor, Chlor oder Brom in Frage.

Die Substituenten $R_2$ und/oder $R_3$ des Phenylringes stellen vorzugsweise Wasserstoff, Halogen, insbesondere Chlor, Nitro, Cyano, Hydroxy, niederes Alkyl, insbesondere Methyl, niederes Alkoxy, insbesondere Methoxy oder Äthoxy, oder niederes Alkyl- oder Alkoxycarbonyl, insbesondere Acetyl, Methoxycarbonyl oder Äthoxycarbonyl, dar. So sind z.B. Verbindungen bevorzugt, worin $R_3$ Wasserstoff ist und $R_2$ Wasserstoff, Halogen, insbesondere Chlor, Nitro, niederes Alkoxy, insbesondere Methoxy, oder niederes Alkylcarbonyl, insbesondere Acetyl, bedeutet und vorzugsweise in 4- oder auch in 2-Stellung angeordnet ist.

Falls $R_4$ für eine Phenylgruppe der Formel a steht, stellen $R_6$ und/oder $R_7$ vorzugsweise Wasserstoff, Halogen, insbesondere Fluor oder Chlor, niederes Alkyl, insbesondere Methyl, oder niederes Alkoxy, insbesondere Methoxy dar. Geeignet sind insbesondere Verbindungen, worin $R_7$ Wasserstoff ist und $R_6$ Wasserstoff oder Halogen, insbesondere Fluor, bedeutet und vorzugsweise in 4- oder auch in 2-Stellung angeordnet ist. Als besonders günstig erweist sich der 4-Fluorphenylrest.

Falls $R_4$ eine Pyridylgruppe der Formel b darstellt, kommt bevorzugt eine 2-Pyridyl-Gruppe in Frage. In der Pyridylgruppe stehen vorzugsweise $R_7$ für Wasserstoff und $R_6$ für Wasserstoff, niederes Alkyl, insbesondere Methyl, oder auch Halogen, insbesondere Chlor. Als besonders günstig erweist sich die 4-Methyl-2-pyridylgruppe.

$R_1$ stellt eine geradkettige, verzweigte oder cyclische Alkylgruppe mit bis zu 6 Kohlenstoffatomen dar. Cyclische Alkylgruppen können 3-6, insbesondere 5-6, Kohlenstoffatome enthalten und umfassen z.B. Cycloalkylgruppen wie Cyclopropyl, Cyclopentyl oder vorzugsweise Cyclohexyl. Nichtcyclische Alkylgruppen können geradkettig oder verzweigt sein und 1-6 Kohlenstoffatome enthalten. Vorzugsweise liegt eine Kettenlänge von 1-4, insbesondere 1-3 Kohlenstoffatomen vor. Beispiele geeigneter nicht cyclischer Alkylgruppen sind Methyl, Äthyl, geradkettige oder verzweigte Propyl- oder Butylgruppen und durch Methyl oder Äthyl substituiertes Butyl. Unter diesen sind geradkettige Alkylreste mit 1-3 Kohlenstoffatomen, insbesondere Methyl oder Äthyl, bevorzugt.

Z steht für eine Alkylenkette mit 2-4 Kohlenstoffatomen, welche geradkettig sein oder eine durch Methyl substituierte Kette darstellen kann, und enthält insbesondere 3-4 Kohlenstoffatome. Vorzugsweise steht Z für eine Propylenkette.

Erfindungsgemäß werden die neuen 5-Alkyl-1--phenyl-2-piperazinoalkylpyrazolin-3-on-Verbindungen der Formel I und deren Säureadditionssalze erhalten, indem man in an sich bekannter Weise

a) Verbindungen der Formel II oder III

(siehe Formeln II und III)

worin $R_1$, $R_2$, $R_3$ und Z obige Bedeutung besitzen, Y einen aminolytisch abspaltbaren Rest und Y' Halogen bedeuten, oder deren Gemische mit Verbindungen der Formel IV

(siehe Formel IV)

worin $R_4$ obige Bedeutung besitzt, umsetzt oder

b) zur Herstellung von Verbindungen der allgemeinen Formel Ia

(siehe Formel Ia)

worin $R_1$, $R_2$, $R_3$ und Z obige Bedeutung besitzen und $R_4'$ für eine 2-Pyridylgruppe der Formel b'

(siehe Formel b')

worin $R_6$ und $R_7$ obige Bedeutung besitzen, steht, Verbindungen der Formel V

(siehe Formel V)

worin $R_1$, $R_2$, $R_3$ und Z obige Bedeutung besitzen, mit Verbindungen der Formel VI

(siehe Formel VI)

worin $R_4'$ obige Bedeutung besitzt und Hal Halogen bedeutet, umsetzt, oder

c) zur Herstellung von Verbindungen der allgemeinen Formel Ib

(siehe Formel Ib)

worin $R_1$, $R_3$ und $R_4$ obige Bedeutung besitzen, $R_2$ die für $R_2$ angegebene Bedeutung mit Ausnahme von Cyano und von eine CO-Gruppe enthaltenden Resten besitzt, und Z' eine Alkylenkette mit 2 oder 3 Kohlenstoffatomen bedeutet, Verbindungen der Formel VII

(siehe Formel VII)

worin $R_1$, $R_2'$, $R_3$, $R_4$ und Z' obige Bedeutung besitzen, reduziert und gewünschtenfalls in erhaltenen Verbindungen der Formel I aus allfälligen Methoxysubstituenten die Hydroxygruppe oder aus allfälligen Alkoxycarbonylsubstituenten die Carboxylgruppe freisetzt und gegebenenfalls freie Verbindungen der Formel I in ihre Säureadditionssalze überführt oder die Säureadditionssalze in die freien Verbindungen der Formel I überführt.

Die Umsetzung von Verbindungen der Formeln II oder III oder deren Gemischen mit Verbindungen der Formel IV gemäß Verfahrensvariante a) kann nach an sich zur Alkylierung von Aminen üblichen Methoden ausgeführt werden.

Die Umsetzung wird zweckmäßigerweise in einem unter den Reaktionsbedingungen inerten organischen Lösungsmittel unter basischen Bedingungen durchgeführt. Als aminolytisch abspaltbare Reste in den Verbindungen der Formel II kommen insbesondere Halogene wie Chlor, Brom oder Jod, vorzugsweise Chlor oder Brom in Frage. Als Beispiele geeigneter Lösungsmittel seien genannt aromatische Kohlenwasserstoffe wie Toluol, Xylol oder Benzol, cyclische Äther wie Dioxan, Dimethylformamid, Sulfolan, Dimethylsulfoxid, Tetramethylharnstoff oder niedere Alkanole, beispielsweise Isopentanol. Die Temperatur kann zwischen Raumtemperatur und 150°C betragen, wobei zweckmäßigerweise erhöhte Temperaturen, z.B. Temperaturen zwischen 50 und 150°C, vorzugsweise Siedetemperatur des Lösungsmittels verwendet werden.

Gewünschtenfalls kann die Umsetzung der Verbindungen der Formeln II oder III mit Verbindungen der Formel IV jedoch auch ohne Lösungsmittel in der Schmelze stattfinden. Zweckmäßig kann die Reaktion unter Zusatz einer organischen oder anorganischen Base durchgeführt werden. Es kann jedoch auch ein Überschuß der Verbindung der Formel IV verwendet und dieser als interne Base benutzt werden. Geeignete anorganische Basen sind insbesondere Alkalimetallcarbonate oder -bicarbonate. Als organische Basen sind tertiäre organische Amine, insbesondere tertiäre Niederalkylamine wie Triäthylamin, 1,4-Dimethylpiperazin oder Pyridin geeignet.

Falls die Verbindungen der Formeln II, III oder IV als Substituenten freie Hydroxy- oder Carboxylgruppen enthalten, werden diese zweckmäßigerweise während der Umsetzung in an sich bekannter Weise mit einer Schutzgruppe versehen. Geeignete nach der Reaktion leicht wieder abspaltbare Schutzgruppen sind z.B. bekannt aus E. McOmie «Protective Groups in Organic Chemistry» Plenum Press 1971. Beispielsweise eignen sich zum Schutze einer Hydroxylfunktion Äther, insbesondere Tetrahydropyranyläther. Zum Schutze der Carboxylfunktion eignen sich niedere Alkylester. Diese Schutzgruppen können nach der Umsetzung in an sich bekannter Weise leicht wieder entfernt werden.

Die Umsetzung von Verbindungen der Formel V mit Verbindungen der Formel VI gemäß Verfahrensvariante b) kann ebenfalls auf an sich bekannte Weise unter zur Alkylierung von Aminen üblichen Bedingungen, beispielsweise den vorstehend für die Umsetzung von Verbindungen der Formel II mit Verbindungen der Formel IV genannten Bedingungen, erfolgen. Die 2-Halogenopyridin-Verbindungen der Formel VI sind zur Umsetzung mit dem Piperazinderivat der Formel V befähigt.

Die Reduktion der Verbindungen der Formel VII gemäß Verfahrensvariante c) kann auf an sich bekannte Weise nach zur Reduktion von Amidgruppen üblichen Verfahren durchgeführt werden. Als Reduktionsmittel eignen sich beispielsweise Hydridreduktionsmittel wie Diboran bzw. Natruimborhydrid in schwach saurem Medium, Lithiumborhydrid oder Lithiumaluminiumhydrid. Die Reduktion mit Natriumborhydrid kann z.B. in einem unter den Reaktionsbedingungen inerten Lösungsmittel in schwach saurem pH-Bereich durchgeführt werden. Als Lösungsmittel können z.B. cyclische oder offene Äther wie Dioxan, Tetrahydrofuran, Äthylenglycoldimethyläther oder Diäthylenglycoldimethyläther eingesetzt werden, es

können auch Dimethylformamid oder niedermolekulare Alkohole verwendet werden. Zur Einstellung des jeweils günstigen pH-Bereichs können organische oder anorganische Säuren wie z.B. Essigsäure, aromatische Sulfonsäuren oder Salzsäure dienen. Die Verfahrensvariante c) eignet sich insbesondere zur Herstellung solcher Verbindungen der Formel I, worin Z eine Butylenkette darstellt.

Eine allfällige Freisetzung der Hydroxygruppe aus einem Methoxysubstituenten kann nach an sich zur Ätherspaltung üblichen Methoden erfolgen. Die Freisetzung der Carboxylgruppe aus einem Alkoxycarbonylsubstituenten kann auf an sich bekannte Weise nach zur Esterhydrolyse üblichen Methoden erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren wie Chlorwasserstoffsäure, Bromwasserstoffsäure, Schwefelsäure, Phosphorsäure oder organischen Säuren wie Methansulfonsäure. Äthansulfonsäure, Benzolsulfonsäure, p-Toluolsulfonsäure, Essigsäure, Milchsäure, Zitronensäure, Bernsteinsäure, Maleinsäure, Fumarsäure, Äpfelsäure, Weinsäure, Benzoesäure, Phenylessigsäure oder Mandelsäure.

Die Verbindungen der Formel I enthalten mehrere basische Zentren und können somit Säureadditionssalze mit mehreren Äquivalenten Säure bilden. Zur Herstellung von pharmazeutischen Zubereitungen eignen sich insbesondere monosaure Salze. Salze, welche mehrere Äquivalente Säure enthalten, können gewünschtenfalls in an sich bekannter Weise in monosaure Salze überführt werden, z.B. durch Überführen in die freie Base und anschließende Umsetzung der Base mit einer äquivalenten Menge Säure. Sofern das monosaure Salz wenig wasserlöslich ist, kann es auch durch vorsichtiges langsames Alkalisieren einer angesäuerten (vorzugsweise pH 4) wäßrigen Lösung von der Base bzw. von deren mehrere Äquivalente Säure enthaltenden Salzen ausgefällt werden.

Verbindungen der Formel I, worin Z eine verzweigte Alkylenkette darstellt, werden bei der Synthese in Form ihrer Racemate erhalten. Unter den Schutz dieser Erfindung fallen die racemischen Gemische wie auch die optisch aktiven Formen dieser Verbindungen. Die optisch aktiven Verbindungen können aus den racemischen Gemischen in an sich bekannter Weise durch Umsetzung mit geeigneten optisch aktiven Säuren wie beispielsweise Weinsäure und anschließende fraktionierte Kristallisation der gewonnenen Salze in ihre optisch aktiven Antipoden aufgetrennt werden.

Verbindungen der Formeln II und III stellen wertvolle Zwischenprodukte für die Herstellung von pharmakologisch aktiven Verbindungen, beispielsweise den Verbindungen der Formel I, dar. Verbindungen der Formeln II und III, worin $R_1$ 2-6 Kohlenstoffatome enthält oder, falls $R_2$ und $R_3$ nicht Wasserstoff bedeutet, auch Methyl ist, sind neu.

Verbindungen der Formeln II und III können nach an sich bekannten Verfahren erhalten werden. So kann man beispielsweise Verbindungen der Formel II, worin Y Halogen bedeutet, und Verbindungen der Formel III erhalten, indem man in situ hergestellte Alkalimetallsalze von 5-Alkyl-1-phenyl-pyrazolin-3-on-Verbindungen der Formel VIII

(siehe Formel VIII)

worin $R_1$, $R_2$ und $R_3$ obige Bedeutung besitzen, mit Verbindungen der Formel IX

(siehe Formel IX)

worin Z, Y und Y' obige Bedeutung besitzen, umsetzt. Y' und Y stellen vorzugsweise Chlor oder Brom dar.

Die Umsetzung erfolgt zweckmäßigerweise in einem unter den Reaktionsbedingungen inerten Lösungsmittel bei Temperaturen zwischen 0°C und Siedetemperatur des Lösungsmittels. Im allgemeinen sind Temperaturen zwischen 0°C und 120°C vorteilhaft. Als Lösungsmittel eignen sich beispielsweise niedere Alkohole wie Methanol, Äthanol, Isopropanol oder Butanole, aber auch aromatische Kohlenwasserstoffe wie Benzol oder Toluol, Dimethylformamid, Sulfolan, Hexamethylphosphortriamid, Tetramethylharnstoff oder cyclische Äther wie z.B. Dioxan oder Tetrahydrofuran.

Als Alkalimetallsalze der Verbindungen der Formel VIII kommen Lithium-, Natrium- oder Kaliumsalze, vorzugsweise das Natriumsalz, in Frage, welche in situ durch Umsetzen der Verbindungen der Formel VIII mit Alkalimetallalkoholaten oder Alkalimetallhydriden erhalten werden.

Bei der Umsetzung werden neben den offenen Alkylierungsprodukten der Formel II auch cyclische Alkylierungsprodukte der Formel III erhalten. Die Verbindungen II und III liegen in dem Reaktionsgemisch in wechselnden Mengenverhältnissen vor in Abhängigkeit von den verwendeten Lösungsmitteln und Alkalimetallverbindungen, der Reaktionszeit und der jeweiligen Bedeutung der einzelnen Substituenten und der Kettenlänge des Gliedes Z. So bilden sich z.B. bei Verwendung eines niederen Alkohols und des entsprechenden Alkalimetallalkoholats und längeren Reaktionszeiten, z.B. 15-35 Stunden, bevorzugt die cyclischen Verbindungen der Formel III, insbesondere wenn Z eine Propylenkette darstellt. Bei Verwendung von Dimethylformamid und Alkalimetallhydriden und geringeren Reaktionszeiten, beispielsweise von 1-15 Stunden, entstehen dagegen vorrangig die Verbindungen der Formel II. Da beide Verbindungen II und III oder deren Gemische in der folgenden Reaktion eingesetzt werden können, ist eine Trennung der beiden Verbindungen vor der weiteren Umsetzung nicht nötig. Selbstverständlich aber können die cyclischen Verbindungen der Formel III von den offenkettigen Produkten der Formel II auf an sich bekannte Weise durch Kristallisation oder

chromatographische Trennverfahren getrennt werden.

Bei der Alkylierung der 5-Alkyl-1-phenyl-pyrazolin-3-on-Verbindungen der Formel VIII mit den Verbindungen der Formel IX werden im allgemeinen Gemische aus den gewünschten N-alkylierten Produkten und den dazu isomeren O-alkylierten Produkten erhalten. Aus diesen Gemischen kann das N-alkylierte Produkt chromatographisch oder durch Kristallisation abgetrennt werden. Die O-alkylierten Nebenprodukte können durch einfaches Erhitzen in die entsprechenden N-alkylierten Produkte der Formel II und/oder die cyclischen Immoniumsalze der Formel III umgelagert werden. Die Umlagerungstemperatur liegt zweckmäßigerweise zwischen 60 und 200°C. Gewünschtenfalls kann die Umlagerung in Gegenwart eines inerten Lösungsmittels zweckmäßigerweise bei Siedetemperatur des Lösungsmittels durchgeführt werden. Geeignete Lösungsmittel sind in dem angegebenen Bereich siedende niedere Alkohole, beispielsweise Methanol, Butanol oder Isopentanol, oder aromatische Kohlenwasserstoffe wie Benzol, Toluol oder Xylol. Für die thermische Umlagerungsreaktion können auch die bei der Alkylierung anfallenden Gemische aus cyclischen Immoniumverbindungen III, N-alkylierten Produkten II und den dazu isomeren O-alkylierten Produkten direkt ohne vorherige Trennung eingesetzt werden.

Verbindungen der Formel V sind in der Literatur bisher noch nicht beschrieben worden und stellen neue wertvolle Zwischenprodukte für die Herstellung von pharmakologisch aktiven Verbindungen, beispielsweise Verbindungen der Formel I, dar.

Verbindungen der Formel V können nach an sich bekannten Methoden erhalten werden, indem man beispielsweise Verbindungen der Formeln II oder III mit einem Überschuß Piperazin umsetzt. Die Umsetzung kann nach an sich zur Alkylierung von Aminen üblichen Methoden, beispielsweise unter den vorstehend für die Umsetzung von Verbindungen der Formeln II oder III mit Verbindungen der Formel IV beschriebenen Bedingungen, durchgeführt werden.

Verbindungen der Formel V können auch aus Verbindungen der Formel X

(siehe Formel X)

worin $R_1$, $R_2$, $R_3$ und Z obige Bedeutung besitzen und Q für eine Aminschutzgruppe steht, erhalten werden, indem man die Aminschutzgruppe auf an sich bekannte Weise abspaltet. Als Aminschutzgruppen kommen die an sich bekannten zum Schutz einer Aminofunktion gebräuchlichen Schutzgruppen, beispielsweise hydrolytisch abspaltbare Acylgruppen oder hydrogenolytisch abspaltbare Benzylgruppen in Frage. Geeignete Schutzgruppen sind z.B. bekannt aus E. McOmie «Protective Groups in Organic Chemistry», Plenum Press, London (1971) S. 44 ff. Insbesondere eignen sich die Formylgruppe und niedere Carbalkoxyschutzgruppen. Diese können in an sich bekannter Weise durch saure oder alkalische Hydrolyse abgespalten werden.

Verbindungen der Formel X können auf an sich bekannte Weise erhalten werden, beispielsweise durch Umsetzung von Verbindungen der Formeln II oder III mit Verbindungen der Formel XI

(siehe Formel XI)

worin Q obige Bedeutung besitzt.

Verbindungen der Formel VII sind in der Literatur bisher noch nicht beschrieben worden und stellen neue wertvolle Zwischenprodukte für die Herstellung von pharmakologisch aktiven Verbindungen, beispielsweise den Verbindungen der Formel I, dar.

Verbindungen der Formel VII können auf an sich bekannte Weise ausgehend von entsprechenden Verbindungen der Formeln II oder III oder deren Gemischen erhalten werden. So können z.B. Verbindungen der Formeln II und/oder III zunächst mit Alkalimetallcyaniden zu Nitrilen der Formel XII

(siehe Formel XII)

worin $R_1$, $R_2'$, $R_3$ und Z' obige Bedeutung besitzen, umgesetzt werden und diese durch saure Hydrolyse in die entsprechenden Carbonsäuren der Formel XIII

(siehe Formel XIII)

worin $R_1$, $R_2$, $R_3$ und Z' obige Bedeutung besitzen, überführt werden und letztere anschließend mit entsprechenden Piperazinverbindungen der Formel IV zu den Amidverbindungen der Formel VII umgesetzt werden.

Die Umsetzung der Verbindungen der Formeln II oder III mit dem Alkalimetallcyanid, vorzugsweise Natriumcyanid erfolgt zweckmäßigerweise in einem unter den Reaktionsbedingungen inerten Lösungsmittel, beispielsweise Dimethylformamid, bei erhöhter Temperatur, vorzugsweise Siedetemperatur des Lösungsmittels. Die Hydrolyse der Nitrile der Formel XIII zu den entsprechenden Carbonsäuren kann nach an sich zur Nitrilverseifung üblichen Methoden sauer, beispielsweise durch Behandeln mit einer wäßrigen Mineralsäure, erfolgen. Die Umsetzung der Carbonsäuren der Formel XIII mit der Piperazinverbindung der Formel IV erfolgt nach an sich zur Amidbildung üblichen Methoden. Zweckmäßigerweise wird die Carbonsäure der Formel XIII zunächst in situ in ein reaktionsfähiges Derivat überführt, beispielsweise durch Umsetzen mit Chlorameisensäureäthylester, und das gebildete reaktive Säurederivat wird anschließend sofort mit dem Piperazin der Formel IV weiter umgesetzt. Die Umsetzung erfolgt vorzugsweise in einem unter den Reaktionsbedingungen inerten Lösungsmittel, beispielsweise einem halogenierten Kohlenwasserstoff wie Chloroform bei Temperaturen zwischen −10°C und Raumtemperatur. Zweckmäßig kann die Reaktion unter Zusatz einer organischen oder anorganischen Base durchgeführt werden. Man kann jedoch auch einen Überschuß der Verbindung der Formel IV verwenden und diesen als interne Base benutzen. Geeignete organische Basen sind tertiäre organische Amine, insbesondere tertiäre niedere Alkylamine wie Triäthylamin.

Die 5-Alkyl-1-phenyl-pyrazolin-3-one der Formel VIII sind bekannt oder können nach an sich bekannten Methoden hergestellt werden. Beispielsweise

können die Verbindungen ausgehend von entsprechenden Acylessigsäureestern und entsprechend substituierten β-Acetyl-phenylhydrazinen nach der von K. Mayer [Ber., 36 (1903), 717] beschriebenen Methode erhalten werden. Ferner können die Verbindungen der Formel VIII auch nach der von L. Lederer beschriebenen Methode [J. Prakt. Chem., 27 (1892), 83] durch Oxidation von entsprechenden 5-Alkyl-1-phenyl-pyrazolin-3-onen erhalten werden, welche ihrerseits durch Umsetzung von entsprechenden β-Halogencarbonsäuren mit entsprechend substituierten Phenylhydrazinen und anschließende Cyclisierung oder durch Umsetzung von entsprechend substituierten Acrylsäureestern oder -amiden mit entsprechend substituierten Phenylhydrazinen (siehe U.S. Patente Nr. 2 688 024 und 2 704 762) hergestellt werden können.

Verbindungen der Formel IV sind bekannt oder können nach an sich bekannten Methoden hergestellt werden, beispielsweise durch Umsetzung von Aminen der Formel XIV

(siehe Formel XIV)

worin $R_4$ obige Bedeutung besitzt, mit Di(2-halogenäthyl)aminen unter zur Alkylierung von Aminen üblichen Bedingungen.

Die Verbindungen der Formel I und ihre pharmakologisch akzeptablen Säureadditionssalze besitzen interessante pharmakologische Eigenschaften, insbesondere ausgeprägte antiallergische Eigenschaften, und zeichnen sich durch ein günstiges Wirkungsprofil sowie gute Verträglichkeit und geringe Toxizität aus. So zeigen die Verbindungen neben einer inhibierenden Wirkung auf durch Antigene induzierte allergische Reaktionen auch eine ausgeprägte Wirkung gegen durch endogene Mediatoren wie Histamin und Serotonin induzierte allergische Hautreaktionen und wirken hemmend gegenüber der zu allergischen Reaktionen führenden Freisetzung endogener Mediatoren aus Mastzellen. Außerdem besitzen die Verbindungen ödemhemmende Eigenschaften.

Die antiallergischen und antiödematösen Eigenschaften der Verbindungen der Formel I können in pharmakologischen Standardtests an Tieren nachgewiesen werden.

Die Verbindungen zeigen beispielsweise eine spezifische hemmende Wirkung in dem nachstehend beschriebenen PCA-Test (passive cutane Anaphylaxie) an der Ratte. Die peripheren Antihistamin- und Antiserotoninwirkungen können ebenfalls in der gleichen Testanordnung durch die Hemmwirkung auf die durch Histamin oder Serotonin induzierten anaphylactoiden Reaktionen der Haut nachgewiesen werden. Die Hemmwirkung auf die anaphylactisch verursachte Freisetzung von Histamin aus den Mastzellen kann z.B. in vitro an isolierten peritonalen Mastzellen nachgewiesen werden.

*Beschreibung der Testmethoden*

1. Akute Toxizität

Die akute 8-Tage Toxizität wird nach einmaliger i.p. Applikation an männlichen Mäusen (Körpergewicht 18 bis 22 g) bestimmt. Die Mortalität wird 8 Tage lang notiert. Die Ergebnisse werden als $LD_{50}$-Werte, das ist die Dosis welche 50% der Tiere tötet, berechnet.

2. Bestimmung der Hemmung der passiven cutanen Anaphylaxie (PCA) und der Hemmung der durch Histamin und der durch Serotonin induzierten anaphylactoiden cutanen Reaktionen

Zur Herstellung des in dem Test verwendeten IgEreichen Antiovoalbuminserums nach der Methode von Lehrer et al [J. Immunol. 116 (1976), 178-182] und Pauwels et al [Ann. Immunol. 203C (1979), 49-58], werden Sprague-Dawley-Ratten durch i.p. Injektion von 1 mg Ovoalbumin und 1 ml Bordetellapertussis Suspension (Vaxicoq®, Merieux $3 \times 10^{10}$ Organismen/ml) sensibilisiert. Nach 20 Tagen erhalten die Tiere eine Injektion von 10 mg Ovoalbumin in der gleichen Weise, jedoch ohne Zusatz. Nach weiteren 4 Tagen werden die Tiere entblutet, und das Blut zentrifugiert. Das so erhaltene Antiserum wird bei −20°C gelagert und nach der Methode von Goose et al [Immunology 16 (1968), 749-760] eingestellt.

Die Bestimmung der Hemmung der passiven cutanen Anaphylaxie und der durch Histamin und der durch Serotonin induzierten anaphylactoiden cutanen Reaktionen wird nach der Methode von Martin und Baggiolini [Naunyn-Schmiedeberg's Archives of Pharmacology 316 (1981), 186-189], wie folgt durchgeführt.

Zur passiven Sensibilisierung gegen Ovoalbumin werden männlichen Sprague-Dawley-Ratten (Körpergewicht 150-165 g) 0,05 ml einer 1:2,5 Verdünnung von IgE-reichem Antiovoalbuminserum in physiologischer Natriumchloridlösung intradermal an einer rasierten Stelle des Rückens injiziert.

24 Stunden nach der Sensibilisierung werden den Ratten Lösungen der Testsubstanzen oral appliziert. Eine Kontrollgruppe erhält zum Vergleich nur das Lösungsmittel.

Zur Auslösung der histamininduzierten und der serotonininduzierten anaphylactoiden Hautreaktionen werden den Tieren 30 Minuten später an einer zweiten rasierten Stelle des Rückens eine Lösung von 40 µg Histamin in 0,05 ml physiologischer Natriumchloridlösung und an einer dritten rasierten Stelle des Rückens eine Lösung von 1 µg Serotonin in 0,05 ml physiologischer Natriumchloridlösung intradermal injiziert.

Sofort anschließend werden den Tieren 1,25 mg (≙ ~ 8 mg/kg) Ovoalbumin zur Auslösung der anaphylactischen Reaktion und 4 mg (≙ ~ 26,4 mg/kg) eines blauen Farbstoffes gelöst in 0,5 ml phosphatgepufferter physiologischer Natriumchloridlösung i.v. appliziert.

Nach 30 Minuten werden die Tiere getötet, die Haut wird entfernt und in den einzelnen blauen Flecken wird die darin enthaltene Farbmenge separat bestimmt. Nach der Methode von Mordelet-Danbrine et al [Therapie 29 (1974), 851-682] wird die Farbe aus den einzelnen Flecken jeweils mit 2,5 ml Dimethylformamid bei einer Temperatur von 37°C während 4 Tagen extrahiert und die jeweils in der Extraktionslösung enthaltene Farbmenge durch spektrophotometrische Messung bestimmt. Als $ED_{50}$-Werte werden diejenigen Dosen der Testsubstanzen

berechnet, welche eine 50%ige Verminderung der Farbkonzentration im Vergleich zu der Kontrollgruppe bewirken.

3. Bestimmung der in vitro Hemmwirkung auf die anaphylactisch bedingte Freisetzung von Histamin aus peritonalen Mastzellen der Ratte

Zur Gewinnung der peritonalen Mastzellen werden Sprague-Dawley-Ratten durch Ätherinhalation getötet und den Tieren sofort nach Eintritt des Atemstillstands 15 ml einer kalten Lösung von 1 mg/ml menschlichem Serumalbumin in phosphatgepufferter Natriumchloridlösung in die Bauchhöhle injiziert, dann wird die peritonale Flüssigkeit entnommen, die Mastzellen daraus durch Zentrifugieren abgetrennt, in phosphatgepufferter Natriumchloridlösung suspendiert und die Suspension auf eine Mastzellenkonzentration von $5 \cdot 10^5$ pro ml eingestellt.

Die Mastzellensuspension wird in Proben von je 100 µl aufgeteilt in Reagenzröhrchen gegeben und jede Probe wird zur passiven Sensibilisierung mit 100 µl einer 1:10 Verdünnung von IgE-reichem Antiovoalbuminserum in physiologischer Natriumchloridlösung (nach der vorstehend beschriebenen Methode hergestellt) versetzt und 30 Minuten bei einer Temperatur von 37°C (Wasserbad) inkubiert. Zur Entfernung von nicht gebundenem IgE werden die Mastzellen anschließend mit 2 ml phosphatgepufferter Natriumchloridlösung gewaschen, zentrifugiert und die überstehende Flüssigkeit wird verworfen. Dann werden die Mastzellen eine Minute lang bei 37°C mit 200 µl einer Lösung der Testsubstanz in phosphatgepufferter Natriumchloridlösung inkubiert. Eine Kontrollgruppe wird nur mit der gleichen Menge testsubstanzfreier phosphatgepufferter Natriumchloridlösung inkubiert.

Zur Auslösung der anaphylactisch bedingten Histaminfreisetzung werden den so behandelten Mastzellen 200 µl einer Lösung von 100 µg/ml Ovoalbumin in phosphatgepufferter Natriumchloridlösung zugesetzt. Nach einer Inkubationszeit von 3 Minuten bei 37°C wird die Reaktion durch schnelles Kühlen in einem Eisbad abgestoppt. Dann wird zentrifugiert, die überstehende Flüssigkeit dekantiert und der Histamingehalt darin ohne weitere Extraktion direkt spektrofluorimetrisch nach der Methode von Shore [J. pharmac. Exp. Ther. 127 (1959), 182-186] bestimmt.

Zur Extraktion des in den Mastzellen des Bodenstands noch enthaltenen restlichen Histamins werden diese mit 2 ml phosphatgepufferter Natriumchloridlösung versetzt und die Suspension im kochenden Wasserbad erwärmt. Anschließend wird zentrifugiert, die überstehende Flüssigkeit dekantiert und darin das aus den Mastzellen extrahierte Resthistamin spektrofluorimetrisch bestimmt.

Für jede Probe wird die Menge des freigesetzten Histamins als Prozentanteil der Gesamthistaminmenge (= Summe aus freigesetztem und in den Mastzellen zurückgebliebenem Histamin) berechnet.

Als $EC_{50}$-Werte werden diejenigen molaren Konzentrationen der Testsubstanzen in der eingesetzten Testlösung berechnet, welche eine 50%ige Hemmung der Histaminfreisetzung im Vergleich zu der Kontrollgruppe bewirken.

Die folgenden Tabellen A und B geben nach den vorstehend beschriebenen Testmethoden erhaltene Ergebnisse wieder. Die für die Verbindungen der Formel I angegebenen Beispielsnummern beziehen sich auf die nachstehenden Herstellungsbeispiele.

TABELLE A

| Testsubstanz Beispiel Nr. | Hemmwirkung gegenüber cutanen allergischen Reaktionen in der Ratte, $ED_{50}$ mg/kg p.o. | | | Akute Toxizität mg/kg Maus i.p. |
|---|---|---|---|---|
| | passive cutane Anaphylaxie | Histamin-induzierte anaphylactoide Reaktion | Serotonin-induzierte anaphylactoide Reaktion | |
| 1 | 2,5 | 4,7 | 2,6 | 117 |
| 2 | 1,9 | 1,75 | 2,7 | 90 |
| 4 | 3,9 | 7,98 | 5,9 | 130 |
| 7 | 6,6 | 7,9 | 13,0 | 178 |
| 11 | 4,65 | 10,1 | 10,2 | 108 |
| 13 | 2,94 | 5,48 | 1,87 | 96 |
| 15 | 1,9 | 4,4 | 1,7 | 90 |
| 17 | 5,0 | 12,6 | 10,8 | 342 |
| 18 | 4,7 | 6,6 | 6,2 | 205 |
| 21 | 7,1 | 5,1 | 8,3 | 252 |
| 23 | 7,85 | 4,7 | 5,8 | 168 |
| 26 | 6,75 | 6,3 | 7,8 | 72,5 |
| 38 | 3,18 | 3,88 | 5,54 | 93 |
| 39 | 5,7 | 9,7 | 2,5 | 91,5 |
| 43 | 2,45 | 5,79 | 3,25 | |
| 44 | 5,4 | 3,15 | 8,9 | 170 |
| 45 | 6,3 | 11 | 8,0 | 240 |
| 46 | 6,3 | 2,8 | 8,0 | 101 |

TABELLE A (Fortsetzung)

| Testsubstanz Beispiel Nr. | Hemmwirkung gegenüber cutanen allergischen Reaktionen in der Ratte, $ED_{50}$ mg/kg p.o. passive cutane Anaphylaxie | Histamin-induzierte anaphylactoide Reaktion | Serotonin-induzierte anaphylactoide Reaktion | Akute Toxizität mg/kg Maus i.p. |
|---|---|---|---|---|
| 47 | 5,96 | 6,8 | 6,8 | 190 |
| 49 | 4,9 | 10,6 | 3,59 | 96 |
| 54 | 4,45 | 5,46 | 0,52 | 53,3 |
| 55 | 7,26 | 11,86 | 11,80 | 135 |
| 56 | 5,7 | 3,85 | 6,29 | >350 |
| 58 | 1,17 | 10 | 1,41 | 95 |

TABELLE B

| Testsubstanz Beispiel Nr. | in vitro Hemmwirkung gegenüber anaphylactisch bedingte Histaminfreisetzung aus Mastzellen der Ratte $EC_{50}$ Mol/l |
|---|---|
| 1 | $5 \cdot 10^{-5}$ |
| 15 | $1,4 \cdot 10^{-5}$ |
| 18 | $23 \cdot 10^{-5}$ |
| 20 | $1,0 \cdot 10^{-5}$ |
| 22 | $4,3 \cdot 10^{-5}$ |
| 23 | $14 \cdot 10^{-5}$ |
| 26 | $3,5 \cdot 10^{-5}$ |
| 30 | $1,8 \cdot 10^{-5}$ |
| 49 | $5,7 \cdot 10^{-5}$ |
| 54 | $12 \cdot 10^{-5}$ |
| 58 | $12 \cdot 10^{-5}$ |
| 60 | $20 \cdot 10^{-5}$ |
| 62 | $6,2 \cdot 10^{-5}$ |

Die antiödematösen Eigenschaften der Verbindungen der Formel I können durch ihre inhibierenden Wirkungen gegenüber durch Carrageenin-Injektionen in der Rattenpfote verursachter lokaler Ödembildung nachgewiesen werden.

Beschreibung der Testmethode zur Bestimmung der Hemmwirkung auf das Carrageenin-Pfotenödem in der Ratte nach der Methode von Winter et al [Proc. Soc. Exp. Biol. Med. 111, (1962), 544-547].

Es werden männliche Wistar-Ratten mit einem Körpergewicht von etwa 120-140 g verwendet. Eine Dosis von 100 mg/kg der Testsubstanz wird suspendiert in einem Volumen von 0,5 ml pro 100 g Körpergewicht einer 1%igen Tylose®-Lösung (= Methylcellulose) mittels einer Schlundsonde per os verabreicht. Eine Kontrollgruppe erhält nur die Tylose-Lösung. Eine Stunde später werden zum Setzen der Entzündung als Irritanz 0,1 ml einer 1%igen Suspension von Carrageenin (Satiagum E®) in isotonischer Kochsalzlösung intraplantar in die rechte Hinterpfote injiziert. In die linke Hinterpfote wird ein gleiches Volumen an isotonischer Kochsalzlösung injiziert. Sowohl vor als auch 3 Stunden nach Applikation des Irritanz wird das Volumen der einzelnen Rattenpfoten plethysmometrisch gemessen und die Pfotenvolumenschwellung nach Carrageeningabe im Vergleich zur nur mit Kochsalzlösung behandelten Pfote bestimmt. Es wird die durch die Testsubstanz bewirkte Hemmung der Ödembildung bei den behandelten Tieren im Vergleich zu den Tieren der unbehandelten Kontrollgruppe in % angegeben.

Die folgende Tabelle C gibt nach der vorstehend beschriebenen Methode mit Verbindungen der Formel I erhaltene Ergebnisse wieder.

TABELLE C

| Testsubstanz Beispiel Nr. | % Hemmung des Carrageneen Pfotenödems an der Ratte bei einer Dosis von 100 mg/kg p.o. |
|---|---|
| 1 | 52,8 |
| 8 | 56,4 |
| 12 | 49,5 |
| 39 | 42,6 |
| 54 | 45,6 |
| 59 | 45,7 |
| 60 | 62,4 |
| 62 | 63,9 |
| 63 | 42,2 |

Aufgrund ihrer antiallergischen Wirkungen und ihrer peripheren Antihistamin- und Antiserotonin-Wirkungen und ihrer ödemhemmenden Eigenschaften eignen sich die Verbindungen der Formel I und ihre pharmakologisch akzeptablen Säureadditionssalze als Antiallergica zur Behandlung allergischer Erkrankungen wie z.B. Bronchialasthma oder allergischer Rhinitis sowie allergisch bedingter Entzündungen.

Als Heilmittel können Verbindungen der Formel I und ihre pharmakologisch verträglichen Salze zusammen mit üblichen pharmazeutischen Hilfsstoffen in galenischen Zubereitungen wie z.B. Tabletten, Kapseln, Suppositorien, Salben, Lösungen oder Sprays enthalten sein. Diese galenischen Zubereitungen können nach an sich bekannten Methoden hergestellt werden unter Verwendung üblicher fester Trägerstoffe wie z.B. Talcum, Milchzucker oder Stärke oder flüssiger Verdünnungsmittel wie z.B. Wasser, fetten Ölen oder flüssigen Paraffinen.

Die Verbindungen der Formel I können in pharma-

zeutischen Gebrauchsformen verabreicht werden, welche 0,5 bis 100 mg, vorzugsweise 0,5 bis 25 mg Aktivsubstanz pro Einzeldosis enthalten. Die zu verwendende Dosierung wird selbstverständlich der zu behandelnden Spezies und den individuellen Erfordernissen angepaßt werden. Parenterale Formulierungen werden im allgemeinen weniger Aktivsubstanz enthalten als Präparate zur oralen Verabreichung.

Die nachfolgenden Beispiele sollen die Herstellung der neuen Verbindungen der Formel I sowie der neuen Zwischenprodukte näher erläutern.

Die Strukturen der neuen Verbindungen wurden durch spektroskopische Untersuchungen, insbesondere durch eine genaue Analyse der IR und NMR-Spektren gesichert.

*Beispiel 1*

5-Äthyl-1-phenyl-2-{3-[4-(4-fluorphenyl)-piperazin-1-yl]-propyl}-pyrazolin-3-on

A) 21 g 5-Äthyl-1-phenylpyrazolin-3-on werden in 250 ml Methanol suspendiert. Zu dieser Suspension wird eine aus 2,7 g Natrium in 50 ml Methanol hergestellte Natriummethylatlösung bei Raumtemperatur unter Rühren zugegeben. Nach 30minütigem Rühren ist eine klare Lösung entstanden. Zu dieser werden 18,5 g 1-Brom-3-chlorpropan tropfenweise zugesetzt. Die Lösung wird sodann 24 Stunden am Rückfluß erhitzt. Dann wird das Lösungsmittel abgedampft, der Rückstand mit 300 ml Methylenchlorid extrahiert, die organische Phase dreimal mit je 30 ml Wasser gewaschen, und die Waschwasser mit 20 ml Methylenchlorid extrahiert. Die Methylenchloridphasen werden vereinigt, über Natriumsulfat getrocknet und filtriert. Die Lösung wird zur Trockne eingedampft und der verbleibende Rückstand mit Aceton und anschließend mit Äthylacetat behandelt. Es werden 7,5 g 2-Äthyl-1-phenyl-6,7-dihydro-1H,5H-pyrazolo(5,1-b)(1,3)-oxazin-8-ium-chlorid mit einem Schmelzpunkt von 203-204 °C erhalten. Weitere 6 g des gleichen Immoniumsalzes werden durch Extraktion des nach Eindampfen der Waschwasserphasen erhaltenen Rückstandes mit Methylenchlorid erhalten. Gesamtausbeute an Immoniumsalz: 13,5 g.

B) Eine Suspension von 6,61 g des unter A beschriebenen Immoniumsalzes, 4,68 g 1-(4-Fluorphenyl)-piperazin und 4,35 g Natriumcarbonat in 188 ml Toluol wird unter Rühren 8 Stunden am Rückfluß erhitzt. Anschließend wird abgekühlt, mit 100 ml Wasser gewaschen, die organische Lösung abgetrennt und mit 100 ml 25%iger Salzsäurelösung extrahiert. Die wäßrige saure Lösung wird durch Zugabe von Natriumhydroxid alkalisch gestellt und die entstandene Base wird mit Methylenchlorid extrahiert. Die organische Phase wird über Natriumsulfat getrocknet, filtriert und das Lösungsmittel unter vermindertem Druck abgezogen. Das zurückbleibende Öl wird aus Isopropyläther/Cyclohexan kristallisiert. Es werden 6 g 5-Äthyl-1-phenyl-2-{3-[4-(4-fluorphenyl)-piperazin-1-yl]-propyl}-pyrazolin-3-on erhalten. Schmelzpunkt: 70-71 °C.

*Beispiel 2*

5-Methyl-1-(4-nitrophenyl)-2-{3-[4-(4-methyl-pyrid-2-yl)-piperazin-1-yl]-propyl}-pyrazolin-3-on

A) 26,5 g 5-Methyl-1-(4-nitrophenyl)-pyrazolin-3-on werden in 200 ml Methanol suspendiert. Eine aus 3 g Natrium und 100 ml Methanol erhaltene Natriummethylatlösung wird tropfenweise zugesetzt. Zu der entstandenen klaren Lösung werden bei Raumtemperatur 20,5 g 1-Brom-3-chlorpropan gegeben. Die Lösung wird 72 Stunden unter Rühren am Rückfluß erhitzt. Nach dem Abkühlen wird zur Aufarbeitung von den Mineralsalzen abfiltriert und die Lösung zur Trockne eingedampft. Der verbleibende Rückstand wird in Methylenchlorid gelöst, nochmals filtriert und das Filtrat wiederum zur Trockne eingedampft. Zu dem Rückstand werden 200 ml Aceton gegeben. Hierbei scheiden sich 17,5 g gelbes 2-Methyl-1-(4-nitrophenyl)-6,7-dihydro-1H,5H-pyrazolo-(5,1-b)(1,3)-oxazin-8-ium-chlorid ab. Aus dem nach Eindampfen der Acetonlösung erhaltenen Rückstand werden nach 2tägigem Kochen in Methanol weitere 4 g desselben Immoniumsalzes erhalten. Gesamtausbeute 21,5 g, Schmelzpunkt: 218-220°C.

B) Eine Suspension von 6 g des unter A) beschriebenen Immoniumsalzes, 5 ml Triäthylamin und 3,9 g 1-(4-Methyl-pyrid-2-yl)-piperazin in 100 ml Toluol wird unter Rühren 10 Stunden am Rückfluß erhitzt. Zur Aufarbeitung wird nach dem Abkühlen das Triäthylaminhydrochlorid durch Filtration entfernt und die Toluollösung mit 100 ml 10%iger Salzsäurelösung extrahiert. Die saure wäßrige Lösung wird durch Zugabe von 30%iger Natriumhydroxidlösung alkalisch gemacht. Hierbei scheidet sich ein Öl ab, welches durch Zusatz von Äthanol zur Kristallisation gebracht wird. Es werden 7 g Rohprodukte erhalten, welche nach Umkristallisation aus Benzol 5,7 g reines 5-Methyl-1-(4-nitrophenyl)-2-{3-[4-(4-methyl-pyrid-2-yl)-piperazin-1-yl]-propyl}-pyrazolin-3-on ergeben. Schmelzpunkt: 160°C.

*Beispiel 3*

5-Methyl-1-phenyl-2-{2-[4-(3,4-dimethoxyphenyl)-piperazin-1-yl]-äthyl}-pyrazolin-3-on

A) Zu einer Suspension von 26,1 g 5-Methyl-1-phenylpyrazolin-3-on in 200 ml Methanol wird eine aus 3,9 g Natrium und 80 ml Methanol erhaltene Natriummethylatlösung zugesetzt. Nach 30minütigem Rühren werden zu der klaren Lösung 43,02 g 1-Brom-2-chloräthan bei Raumtemperatur zugesetzt. Das Reaktionsgemisch wird 15 Stunden am Rückfluß erhitzt. Anschließend wird das Lösungsmittel unter vermindertem Druck abgezogen. Der verbleibende Rückstand wird in 250 ml Methylenchlorid und 100 ml Wasser gelöst. Die organische Phase wird abgetrennt, über Natriumsulfat getrocknet, filtriert und das Lösungsmittel unter vermindertem Druck abgedampft. Der Rückstand wird in Äthyläther gelöst und die Lösung zur Entfernung von verbliebenem Ausgangsmaterial filtriert. Dann wird das Lösungsmittel eingedampft und der Rückstand (27 g) wird in 100 ml Butanol 8 Stunden am Rückfluß gekocht. Anschließend wird das Lösungsmittel im Vakuum abgedampft und der Rückstand durch Säulenchromatographie über Kieselgel gereinigt. Es werden 20 g 2-(2-Chloräthyl)-5-methyl-1-phenyl-pyrazolin-3-on als Öl erhalten.

B) 3,71 g 2-(2-Chloräthyl)-5-methyl-1-phenyl-pyrazolin-3-on werden in 120 ml Toluol mit 3,5 g 1-(3,4-Dimethoxyphenyl)piperazin, 2,65 g Kaliumcarbonat und 0,5 g Kaliumjodid unter Rühren 24 Stunden am Rückfluß erhitzt. Nach dem Abkühlen wird die Toluolphase mit 25%iger wäßriger Salzsäurelösung extrahiert. Die wäßrige Lösung wird durch Zusatz von 30%iger Natriumhydroxidlösung alkalisch gestellt und dann mit 100 ml Methylenchlorid extrahiert. Die Methylenchloridphase wird über Natriumsulfat getrocknet, filtriert und das Lösungsmittel unter vermindertem Druck entfernt. Der verbleibende Rückstand wird über eine Kieselgelsäule gereinigt. Es werden 2,5 g der reinen Titelverbindung als Öl erhalten. Zur Überführung in ihr Salz wird die Base in wenig Isopropanol gelöst und die Lösung wird mit einer einen 10% Überschuß an HCl entsprechenden Menge einer 2,15 N Lösung von HCl in Isopropanol unter Kühlung versetzt. Das gebildete Trihydrochlorid wird abgesaugt und aus Isopropanol kristallisiert. Es werden 2,3 g 5-Methyl-1-phenyl-2-{2-[4-(3,4-dimethoxyphenyl)-piperazin-1-yl]-äthyl}-pyrazolin-3-ontrihydrochlorid erhalten. Schmelzpunkt: 140 bis 150°C.

*Beispiel 4*

5-Methyl-1-phenyl-2-{4-[4-(4-methyl-pyrid-2-yl)-piperazin-1-yl]-butyl}-pyrazolin-3-on

A) 87 g 5-Methyl-1-phenylpyrazolin-3-on werden in 300 ml Methanol suspendiert. Eine aus 13 g Natrium in 300 ml Methanol erhaltene Natriummethylatlösung wird unter Rühren zu der Suspension gegeben. Zu der entstandenen klaren Lösung werden nach 15 Minuten 87 g 1-Brom-3-chlorpropan tropfenweise zugegeben. Die Lösung wird 24 Stunden am Rückfluß erhitzt. Nach dem Abkühlen werden die Mineralsalze abfiltriert, das Lösungsmittel sodann im Vakuum abdestilliert und der verbleibende Rückstand in 500 ml Methylenchlorid gelöst. Die organische Lösung wird mit 100 ml Wasser gewaschen. Das Waschwasser wird wiederum dreimal mit je 100 ml Methylenchlorid extrahiert und die vereinigten Methylenchloridphasen werden über Natriumsulfat getrocknet und filtriert. Nach Abdampfen des Lösungsmittels im Vakuum werden 98 g Rückstand erhalten. Dieser Rückstand wird in 200 ml Aceton suspendiert und die Suspension wird 5 Minuten am Rückfluß erhitzt und dann unter Rühren abgekühlt. Das kristallin anfallende 2-Methyl-1-phenyl-6,7-dihydro-1H,5H-pyrazolo(5,1-b)(1,3)oxazin-8-ium-chlorid wird abfiltriert. Ferner werden die Waschwasser zur Trockne eingedampft und der erhaltene Rückstand wird mit 400 ml Methylenchlorid extrahiert. Die Methylenchloridlösung wird von den Mineralsalzen abfiltriert, das Lösungsmittel unter vermindertem Druck abdestilliert und der verbleibende Rückstand mit kochendem Aceton behandelt. Es wird eine weitere Menge desselben Immoniumsalzes erhalten. Gesamtausbeute 78 g, Schmelzpunkt: 222°C.

B) 25,07 g des unter A) beschriebenen Immoniumsalzes werden mit 5 g Natriumcyanid in 500 ml Dimethylformamid 6 Stunden unter Rühren am Rückfluß erhitzt. Anschließend wird das Lösungsmittel unter vermindertem Druck entfernt, der Rückstand wird in 200 ml Methylenchlorid und 100 ml Wasser gelöst und die organische Phase abgetrennt und über Natriumsulfat getrocknet. Nach Eindampfen des Lösungsmittels verbleiben 19 g rohes 2-(3-Cyanopropyl)-5-methyl-1-phenylpyrazolin-3-on als öliger Rückstand. Sie werden ohne weitere Reinigung in der nachfolgenden Reaktion eingesetzt.

C) 19 g des vorgehend erhaltenen rohen Nitrils werden in 234 ml 33%iger wäßriger Salzsäure 6 Stunden am Rückfluß erhitzt. Nach dem Abkühlen wird so lange Natriumcarbonat zu der wäßrigen Phase zugegeben, bis die Mischung alkalisch reagiert, und dann wird die Mischung mit Methylenchlorid extrahiert. Die wäßrige Phase wird abgetrennt, mit konzentrierter Salzsäure bis auf pH 2 angesäuert und die ausgefallene rohe Carbonsäure wird abgesaugt und mit Wasser gewaschen. Nach Umkristallisation aus Wasser werden 13,5 g der reinen 4-[(5-Methyl-1-phenyl-3-oxo)-pyrazolin-2-yl]-buttersäure, Schmelzpunkt: 145°C, erhalten.

D) Zu einer Mischung von 13 g der vorgehend erhaltenen Carbonsäure und 11,67 ml Triäthylamin in 242 ml Chloroform wird bei 0°C eine Lösung von 8,2 g Chlorameisensäureäthylester in 42 ml Chloroform tropfenweise innerhalb von 30 Minuten zugegeben. Bei der gleichen Temperatur wird dann eine Lösung von 9,4 g 1-(4-methyl-pyrid-2-yl)-piperazin und 11,67 ml Triäthylamin in 42 ml Chloroform tropfenweise innerhalb 30 Minuten zugesetzt. Die Lösung wird sich auf Raumtemperatur erwärmen gelassen und 2 Stunden bei Raumtemperatur gerührt. Zur Aufarbeitung werden 250 ml Wasser zugesetzt, die organische Phase abgetrennt, über Natriumsulfat getrocknet und das Lösungsmittel abdestilliert. Es werden 30 g öliges Rohprodukt als Rückstand erhalten. Nach Waschen des Rohproduktes mit Diäthyläther werden 24 g öliges 5-Methyl-1-phenyl-2-{3-[4-(4-methyl-pyrid-2-yl)-piperazin-1-yl-carbonyl]-propyl}-pyrazolin-3-on erhalten.

E) 16 g des vorstehend erhaltenen Amids werden in 370 ml Diäthylenglycol-dimethyläther gegeben und dieser Mischung werden unter Rühren bei einer Temperatur von unter 10°C 14,5 g Natriumborhydrid innerhalb 30 Minuten zugesetzt. Bei der gleichen Temperatur werden innerhalb weiterer 30 Minuten 22 ml Essigsäure tropfenweise zugesetzt. Die Lösung wird sodann 6 Stunden am Rückfluß erhitzt. Nach Abkühlen auf Raumtemperatur wird die Mischung mit Wasser hydrolysiert. Zu der erhaltenen Suspension werden 150 ml 50%ige Salzsäurelösung unter Rühren gegeben. Die Lösung wird 4 Stunden bei Raumtemperatur gerührt. Anschließend wird das Wasser abgedampft und der Rückstand mit einer Wasser/Toluol-Mischung extrahiert. Die wäßrige Phase wird abgetrennt, durch Zusatz von Natriumcarbonat alkalisch gestellt und die gebildete Titelbase mit Methylenchlorid extrahiert. Der nach Abdampfen des Lösungsmittels verbleibende Rückstand wird chromatographisch über Kieselgel gereinigt.

Es werden 4,8 g 5-Methyl-1-phenyl-2-{4-[4-(4-methyl-pyrid-2-yl)-piperazin-1-yl]-butyl}-pyrazolin-3-on erhalten. Die Base wird in ihr Trihydrochlorid überführt und dieses aus Isopropanol kristallisiert. Schmelzpunkt: 200°C, Ausbeute: 4,0 g.

*Beispiel 5*

5-Methyl-1-phenyl-2-{3-[4-(4-methyl-pyrid-2-yl)-piperazin-1-yl]-propyl}-pyrazolin-3-on

A) 25 g 2-Methyl-1-phenyl-6,7-dihydro-1H,5H-pyrazolo-(5,1-b)(1,3)-oxazin-8-ium-chlorid (hergestellt analog Beispiel 4 A), 12 g N-formylpiperazin und 33,2 g Kaliumcarbonat werden in 300 ml Toluol 16 Stunden unter Rühren am Rückfluß erhitzt. Nach Abkühlen wird von den Mineralsalzen abfiltriert und die Toluollösung zur Trockne eingedampft. Es werden 30 g 5-Methyl-1-phenyl-2-[3-(4-formylpiperazin-1-yl)-propyl]-pyrazolin-3-on als öliger Rückstand erhalten.

B) Die vorstehend erhaltene Formylverbindung wird in 600 ml 10%iger wäßriger Salzsäure 9 Stunden am Rückfluß erhitzt. Anschließend wird die Reaktionslösung durch Zugabe von Ammoniumhydroxid neutralisiert, eingedampft und mit 200 ml absolutem Äthanol extrahiert. Das Lösungsmittel wird unter vermindertem Druck abdestilliert und es werden 18 g 5-Methyl-1-phenyl-2-[3-(piperazin-1-yl)-propyl]-pyrazolin-3-on-hydrochlorid erhalten.

C) 3,4 g des vorstehend erhaltenen Hydrochlorids werden mit 1,7 g 2-Brom-4-methylpyridin und 3 g Triäthylamin in 80 ml Toluol 24 Stunden am Rückfluß erhitzt. Anschließend wird abgekühlt, von den gebildeten Salzen abfiltriert, und die Toluolphase mit 50 ml 25%iger wäßriger Salzsäure extrahiert. Die wäßrige Phase wird durch Zusatz von 30%iger Natriumhydroxidlösung alkalisch gestellt und mit Methylenchlorid extrahiert. Die Methylenchloridphase wird getrocknet und zur Trockne eingedampft. Die als Rückstand verbleibende Titelbase wird aus Diäthyläther umkristallisiert. Es werden 2,5 g 5-Methyl-1-phenyl-2-{3-[4-(4-methyl-pyrid-2-yl)-piperazin-1-yl]-propyl}-pyrazolin-3-on erhalten. Schmelzpunkt: 107-108°C.

*Beispiel 6*

5-Methyl-1-(4-carboxyphenyl)-2-{3-[4-(4-methyl-pyrid-2-yl)-piperazin-1-yl]-propyl}-pyrazolin-3-on

11 g 5-Methyl-1-(4-methoxycarbonylphenyl)-2-{3-[4-(4-methyl-pyrid-2-yl)-piperazin-1-yl]-propyl}-pyrazolin-3-on-hydrochlorid [siehe Beispiel Nr. 48, hergestellt analog Beispiel 2 A) und B) ausgehend von 5-Methyl-1-(4-methoxycarbonylphenyl)pyrazolin-3-on] werden in 100 ml 85%igem Äthanol gelöst und die Lösung unter Rühren bei Raumtemperatur mit 5 g Kaliumhydroxid versetzt. Nach Beendigung der Reaktion wird das Reaktionsgemisch durch Zugabe von reiner Salzsäure neutralisiert und das Äthanol abdestilliert. Die verbleibende wäßrige Lösung wird mit Chlorwasserstoff auf pH 2 angesäuert, dann wird das Wasser abdestilliert und der Rückstand in Äthanol gelöst. Die ausgefallenen Mineralsalze werden abgesaugt und das Filtrat gekühlt, wobei das Dihydrochlorid der Titelverbindung auskristallisiert.

Es werden 6,3 g 5-Methyl-1-(4-carboxyphenyl)-2-{3-[4-(4-methyl-pyrid-2-yl)-piperazin-1-yl]-propyl}-pyrazolin-3-on-dihydrochlorid erhalten. Fp.: 205 bis 225°C.

*Beispiel 7*

5-Methyl-1-(4-hydroxyphenyl)-2-{3-[4-(4-methyl-pyrid-2-yl)-piperazin-1-yl]-propyl}-pyrazolin-3-on

2 g 5-Methyl-1-(4-methoxyphenyl)-2-{3-[4-(4-methyl-pyrid-2-yl)-piperazin-1-yl]-propyl}-pyrazolin-3-on [siehe Beispiel Nr. 47, hergestellt analog Beispiel 2 A) und B) ausgehend von 5-Methyl-1-(4-methoxyphenyl)-pyrazolin-3-on] werden zu durch Mischen von 9,1 ml Pyridin mit 10 ml konzentrierter Salzsäure und anschließende Verdampfung von Wasser erhaltenem Pyridiniumchlorid bei einer Temperatur von 120°C gegeben. Die Mischung wird bis auf eine Temperatur von 210°C während 30 Minuten erhitzt. Anschließend wird auf Raumtemperatur abgekühlt, 20 ml Wasser zu der Reaktionsmischung gegeben, mit einer Natriumhydroxidlösung neutralisiert und das gebildete Öl mit Chloroform extrahiert. Der Chloroformextrakt wird über Natriumsulfat getrocknet und das Lösungsmittel wird abdestilliert. Nach Zugabe von Diäthyläther zu dem Rückstand kristallisiert die Titelbase aus.

Es werden 0,7 g 5-Methyl-1-(4-hydroxyphenyl-2-{3-[4-(4-methyl-pyrid-2-yl)-piperazin-1-yl]-propyl}-pyrazolin-3-on erhalten. Fp.: 170°C.

Nach den in den vorstehenden Beispielen beschriebenen Verfahren können auch die in der nachfolgenden Tabelle 1 aufgeführten 5-Alkyl-1-phenyl-2-piperazinoalkylpyrazolin-3-on-Verbindungen der Formel I aus entsprechenden Verbindungen der Formeln II, III,V oder VII hergestellt werden.

Nach den in den Beispielen 1 A), 2 A) und 4 A) beschriebenen Verfahren können die in der nachfolgenden Tabelle 2 aufgeführten Immoniumsalze der Formel III aus den entsprechenden 5-Alkyl-1-phenyl-pyrazolin-3-onen der Formel VIII hergestellt werden.

TABELLE 1

| Beispiel Nr. | $R_1$ | $R_2$ | $R_3$ | Z | $R_4$ | Salz | Fp. °C |
|---|---|---|---|---|---|---|---|
| 8 | $CH_3$ | H | H | n-$C_3H_6$ | 4-F-phen | 2,7HCl | 142 |
| 10 | $CH_3$ | H | H | n-$C_3H_6$ | 2-$CH_3O$-phen | 3HCl | 151 |
| 11 | $CH_3$ | H | H | n-$C_3H_6$ | phen | 2HCl | 138-144 |
| 12 | n-$C_3H_7$ | H | H | n-$C_3H_6$ | 4-F-phen | 2,9HCl | 140-142 |
| 13 | n-$C_3H_7$ | H | H | n-$C_3H_6$ | 4-$CH_3$-2-pyr | 3,8HCl | 164 |
| 15 | Cyclohex | H | H | n-$C_3H_6$ | 4-$CH_3$-2-pyr | 4HCl | 150 |
| 17 | $CH_3$ | 2-$CH_3OCO$ | H | n-$C_3H_6$ | 4-$CH_3$-2-pyr | 3HCl | 160 |
| 18 | $CH_3$ | 4-Cl | H | n-$C_3H_6$ | 4-$CH_3$-2-pyr | 3HCl | 168-172 |
| 19 | $CH_3$ | 2,5-di-Cl | | n-$C_3H_6$ | 4-$CH_3$-2-pyr | 2,5HCl | 130 |
| 20 | $CH_3$ | 4-$CH_3$ | 2-Cl | n-$C_3H_6$ | 4-$CH_3O$-phen | Ba | 110-112 |
| 21 | $CH_3$ | 2-$CH_3O$ | H | n-$C_3H_6$ | 4-$CH_3$-2-pyr | 3HCl | 165-170 |
| 22 | n-$C_3H_7$ | 3,4-di-Cl | | n-$C_3H_6$ | 4-F-phen | HCl | 172-180 |
| 23 | $C_2H_5$ | 4-Cl | H | n-$C_3H_6$ | 4-$CH_3$-2-pyr | 3HCl | 200-205 |
| 24 | $CH_3$ | H | H | n-$C_3H_6$ | 6-$CH_3O$-2-pyr | 2HCl | 162 |
| 26 | n-$C_6H_{13}$ | H | H | n-$C_3H_6$ | 4-$CH_3$-2-pyr | 3HCl | 176 |
| 27 | Cyclohex | H | H | n-$C_3H_6$ | 4-Cl-phen | 2HCl | 150 |
| 29 | $CH_3$ | 4-$NO_2$ | H | n-$C_3H_6$ | 2-pyr | 3HCl | 135-145 |
| 30 | $CH_3$ | 4-$C_2H_5O$ | H | n-$C_3H_6$ | 3-Cl-6-$CH_3$-phen | Ba | 119 |
| 31 | $(CH_3)_2$-CH-$CH_2$ | H | H | n-$C_3H_6$ | 2-F-phen | 2HCl | 130-135 |
| 32 | $CH_3$ | H | H | n-$C_3H_6$ | 5-$CH_3$-2-pyr | Ba | 115 |
| 33 | $CH_3$ | H | H | $CH_2$-CH-$CH_2$<br>I<br>$CH_3$ | 4-$CH_3$-2-pyr | 3HCl | 164-168 |
| 34 | $CH_3$ | 3-Br | H | n-$C_3H_6$ | 4-$CH_3$-2-pyr | 3,6HCl | 168 |
| 35 | $CH_3$ | 4-$CH_3OCO$ | H | n-$C_3H_6$ | 4-F-phen | HCl | 157-163 |
| 36 | $CH_3$-$CH_2$-CH<br>I<br>$CH_3$ | H | H | n-$C_3H_6$ | 5-Cl-2-pyr | 2,7HCl | 160 |
| 37 | $C_2H_5$ | H | H | n-$C_3H_6$ | 4-$CH_3$-2-pyr | 3HCl | 184 |
| 38 | $C_2H_5$ | 4-$NO_2$ | H | n-$C_3H_6$ | 4-$CH_3$-2-pyr | Ba | 113 |
| 39 | $CH_3$ | 4-$NO_2$ | H | n-$C_3H_6$ | 4-F-phen | 2HCl<br>Ba | 170-180<br>70- 71 |
| 40 | $CH_3$ | 2-F | H | $CH_2$-$CH_2$-CH<br>I<br>$CH_3$ | 3-Cl-phen | Ba | öl |
| 42 | $CH_3$ | 2-F | H | CH-$CH_2$-$CH_2$<br>I<br>$CH_3$ | 3-Cl-phen | Ba | öl |
| 43 | Cycloprop | H | H | n-$C_3H_6$ | 4-F-phen | Ba | 127-128 |
| 44 | $CH_3$ | 4-$CH_3CO$ | H | n-$C_3H_6$ | 4-$CH_3$-2-pyr | 3HCl | 210 |
| 45 | $CH_3$ | H | H | n-$C_3H_6$ | 2-pyr | 3HCl·1$H_2O$ | 130 |
| 46 | $CH_3$ | 4-CN | H | n-$C_3H_6$ | 4-$CH_3$-2-pyr | 2,4HCl | 200-210 |
| 47 | $CH_3$ | 4-$CH_3O$ | H | n-$C_3H_6$ | 4-$CH_3$-2-pyr | 2,6HCl | 200-205 |
| 48 | $CH_3$ | 4-$CH_3OCO$ | H | n-$C_3H_6$ | 4-$CH_3$-2-pyr | HCl·0,5$H_2O$ | 115-125 |
| 49 | Cyclohex | H | H | n-$C_3H_6$ | 4-F-phen | 2,4HCl | 160-170 |

TABELLE 1 (Fortsetzung)

| Beispiel Nr. | $R_1$ | $R_2$ | $R_3$ | Z | $R_4$ | Salz | Fp. °C |
|---|---|---|---|---|---|---|---|
| 50 | $C_2H_5$ | H | H | $CH_2$-CH-$CH_2$<br>I<br>$CH_3$ | 4-F-phen | Ba | 129-130 |
| 51 | Cyclohex | H | H | $CH_2$-CH-$CH_2$<br>I<br>$CH_3$ | 4-$CH_3$-2-pyr | Ba | 80- 90 |
| 52 | $CH_3$ | H | H | n-$C_4H_8$ | 2,6-di-$CH_3$-phen | 2HCl | 182 |
| 53 | $CH_3$ | 4-$COOC_2H_5$ | H | n-$C_3H_6$ | 4-$CH_3$-2-pyr | HCl·0,5$H_2O$ | 169-170 |
| 54 | $C_2H_5$ | 4-$NO_2$ | H | n-$C_3H_6$ | 4-F-phen | 1,2HCl | 185-190 |
| 55 | $CH_3$ | 4-$CH_3$ | H | n-$C_3H_6$ | 4-$CH_3$-2-pyr | 1,1HCl | 190-195 |
| 56 | $CH_3$ | 3,4-di-$OCH_3$ | | n-$C_3H_6$ | 4-$CH_3$-2-pyr | 2,6HCl·0,3$H_2O$ | 205-210 |
| 58 | Cyclopent | H | H | n-$C_3H_6$ | 4-$CH_3$-2-pyr | 2,7HCl | 162-164 |
| 59 | $CH_3$ | 4-$COCH_3$ | H | n-$C_3H_6$ | 4-F-phen | Ba | 107-110 |
| 60 | $CH_3$ | 4-CN | H | n-$C_3H_6$ | 4-F-phen | Ba | 116-117 |
| 61 | $CH_3$ | 4-$OCH_3$ | H | n-$C_3H_6$ | 4-F-phen | Ba | 132-133 |
| 62 | $CH_3$ | 4-Cl | H | n-$C_3H_6$ | 4-F-phen | Ba | 84- 86 |
| 63 | Cyclopent | H | H | n-$C_3H_6$ | 4-F-phen | 1,8HCl | 140-150 |

pyr = Pyridyl, phen = Phenyl, Cyclohex = Cyclohexyl, Cycloprop = Cyclopropyl, Cyclopent = Cyclopentyl, HCl = Hydrochlorid, öl = ölig, Ba = Base, Fu = Monofumarate

TABELLE 2

| Beispiel Nr. | $R_1$ | $R_2$ | $R_3$ | Z | Y | Fp. °C |
|---|---|---|---|---|---|---|
| 12A | n-$C_3H_7$ | H | H | n-$C_3H_6$ | Cl | 175-176 |
| 15A | Cyclohex | H | H | n-$C_3H_6$ | Cl | 246 |
| 17A | $CH_3$ | 2-$CH_3OCO$ | H | n-$C_3H_6$ | Cl | 218-220 |
| 18A | $CH_3$ | 4-Cl | H | n-$C_3H_6$ | Cl | 188 |
| 18A' | $CH_3$ | 4-Cl | H | n-$C_3H_6$ | Br | 198-200 |
| 19A | $CH_3$ | 2,5-di-Cl | | n-$C_3H_6$ | Cl | 232 |
| 20A | $CH_3$ | 2-Cl-4-$CH_3$ | | n-$C_3H_6$ | Cl | 212-213 |
| 21A | $CH_3$ | 2-$CH_3O$ | H | n-$C_3H_6$ | Cl | 205-206 |
| 22A | n-$C_3H_7$ | 3,4-di-Cl | | n-$C_3H_6$ | Cl | 248-250 |
| 23A | $C_2H_5$ | 4-Cl | H | n-$C_3H_6$ | Cl | 168 |
| 26A | n-$C_6H_{13}$ | H | H | n-$C_3H_6$ | Cl | 118 |
| 30A | $CH_3$ | 4-$C_2H_5O$ | H | n-$C_3H_6$ | Cl | 144 |
| 31A | $(CH_3)_2$-CH-$CH_2$ | H | H | n-$C_3H_6$ | Cl | 192 |
| 33A | $CH_3$ | H | H | $CH_2$-CH-$CH_2$<br>I<br>$CH_3$ | Cl | 176 |
| 34A | $CH_3$ | 3-Br | H | n-$C_3H_6$ | Cl | 200 |
| 35A | $CH_3$ | 4-$CH_3OCO$ | H | n-$C_3H_6$ | Cl | 184-186 |
| 36A | $CH_3$-$CH_2$-CH<br>I<br>$CH_3$ | H | H | n-$C_3H_6$ | Cl | 214-215 |
| 38A | $C_2H_5$ | 4-$NO_2$ | H | n-$C_3H_6$<br>I<br>$CH_3$ | Cl | 205-210 |
| 43A | Cycloprop | H | H | n-$C_3H_6$ | Cl | 148-150 |
| 44A | $CH_3$ | 4-$CH_3CO$ | H | n-$C_3H_6$ | Cl | 177-188 |
| 46A | $CH_3$ | 4-CN | H | n-$C_3H_6$ | Cl | 120-130 |

TABELLE 2 (Fortsetzung)

| Beispiel Nr. | $R_1$ | $R_2$ | $R_3$ | Z | Y | Fp. °C |
|---|---|---|---|---|---|---|
| 47A | $CH_3$ | 4-$CH_3O$ | H | n-$C_3H_6$ | Cl | 203 |
| 50A | $C_2H_5$ | H | H | $CH_2$-CH-$CH_2$<br>I<br>$CH_3$ | Cl | 144-145 |
| 51A | Cyclohex | H | H | $CH_2$-CH-$CH_2$<br>I<br>$CH_3$ | Cl | 220-222 |
| 55A | $CH_3$ | 4-$CH_3$ | H | n-$C_3H_6$ | Cl | 207-208 |
| 56A | $CH_3$ | 3,4-di-$OCH_3$ | | n-$C_3H_6$ | Cl | ha,-140 |
| 58A | Cyclopent | H | H | n-$C_3H_6$ | Cl | 214-215 |

Cyclohex = Cyclohexyl, Cycloprop = Cyclopropyl, Cyclopent = Cyclopentyl, ha = halbfest

In der nachstehenden Tabelle 3 sind die als Ausgangsprodukte für die Verbindungen der Formel III nach aus der Literatur bekannten Methoden hergestellten neuen 5-Alkyl-1-phenyl-pyrazolin-3-one der Formel VIII aufgeführt.

TABELLE 3

| Substanz Nr. | $R_1$ | $R_2$ | $R_3$ | Fp. °C |
|---|---|---|---|---|
| 101 | $C_2H_5$ | H | H | 143-144 |
| 102 | n-$C_3H_7$ | H | H | 136-138 |
| 103 | $(CH_3)_2$-CH-$CH_2$ | H | H | 180 |
| 104 | $CH_3$-$CH_2$-CH<br>I<br>$CH_3$ | H | H | 166-167 |
| 105 | $(CH_3)_3C$ | H | H | 144 |
| 106 | n-$C_6H_{13}$ | H | H | 130 |
| 107 | Cycloprop | H | H | 148 |
| 108 | Cyclohex | H | H | 243 |
| 109 | $CH_3$ | 4-$CH_3CO$ | H | 216 |
| 110 | $CH_3$ | 4-$C_2H_5O$ | H | 170-171 |
| 111 | $CH_3$ | 4-$C_2H_5OCO$ | H | 211 |
| 112 | $CH_3$ | 3-Br | H | 205 |
| 113 | $CH_3$ | 3-$CF_3$ | H | 165 |
| 114 | $CH_3$ | 2-F | H | 201 |
| 115 | $CH_3$ | 2-Cl-4-$CH_3$ | | 209-210 |
| 116 | $C_2H_5$ | 4-Cl | H | 198 |
| 117 | $C_2H_5$ | 4-$NO_2$ | H | 264 |
| 119 | n-$C_3H_7$ | 3,4-di-Cl | | 183-184 |
| 120 | $CH_3$ | 4-CN | H | 255 |
| 121 | $CH_3$ | 4-$CH_3O$ | H | 195-200 |
| 122 | Cyclopent | H | H | 212-213 |
| 123 | $CH_3$ | 3,4-di-$OCH_3$ | | 160-162 |

Cyclohex = Cyclohexyl, Cyclopent = Cyclopentyl, Cycloprop = Cyclopropyl

*Beispiel I* - Tabletten

Man stellt Tabletten in folgender Zusammensetzung pro Tablette her:

| | |
|---|---|
| 5-Äthyl-1-phenyl-2-{3-[4-(4-fluorphenyl)-piperazin-1-yl]-propyl}-pyrazolin-3-on | 15 mg |
| Maisstärke | 60 mg |
| Milchzucker | 140 mg |
| Gelatine (als 10%ige Lösung) | 6 mg |

Der Wirkstoff, die Maisstärke und der Milchzucker werden mit der 10%igen Gelatine-Lösung eingedickt, die Paste wird zerkleinert und das entstandene Granulat wird auf ein geeignetes Blech gebracht und bei 45°C getrocknet. Das getrocknete Granulat wird durch eine Zerkleinerungsmaschine geleitet und in einem Mixer mit weiteren folgenden Hilfsstoffen gemischt:

| | |
|---|---|
| Talkum | 5 mg |
| Magnesiumstearat | 5 mg |
| Maisstärke | 9 mg |

und sodann zu Tabletten zu 240 mg verpreßt.

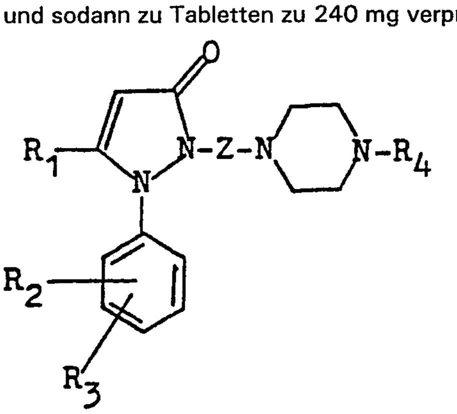

I

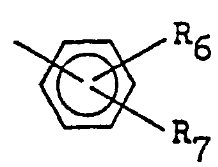

a)

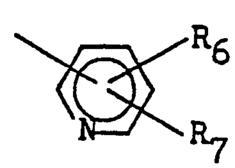

b)

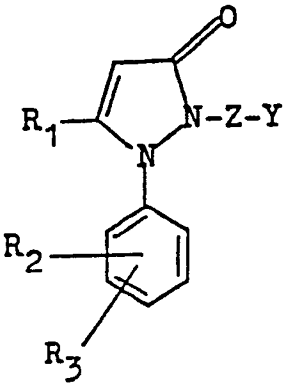

II

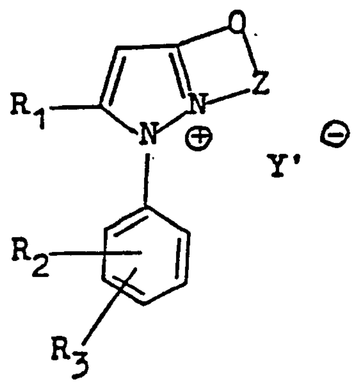

III

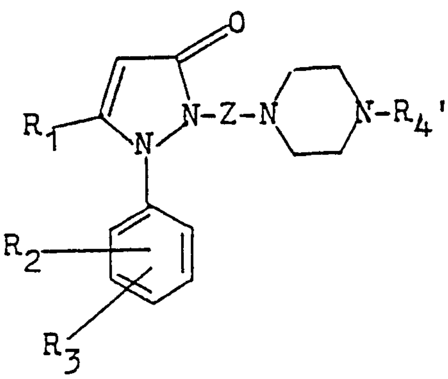

Ia

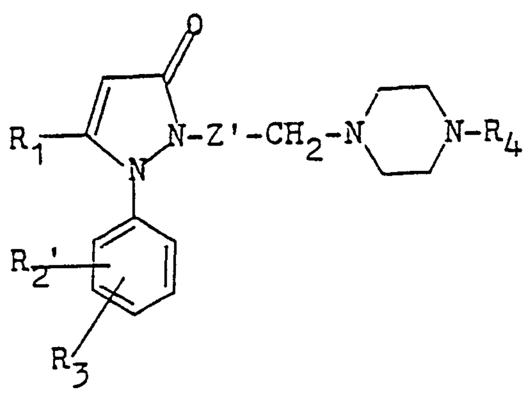

Ib

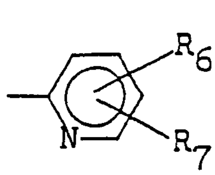

b'

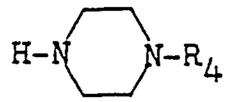

IV

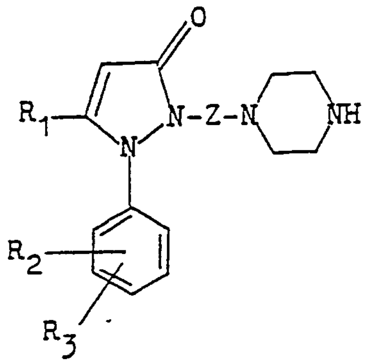

V

Hal-$R_4'$ VI

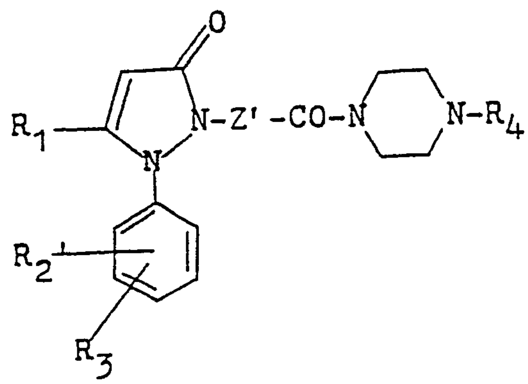

VII

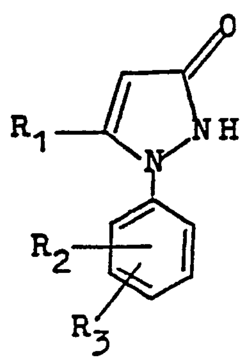

VIII

Y-Z-Y' IX

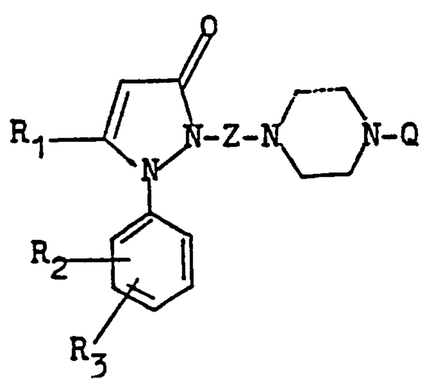

X

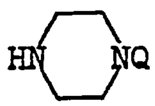

XI

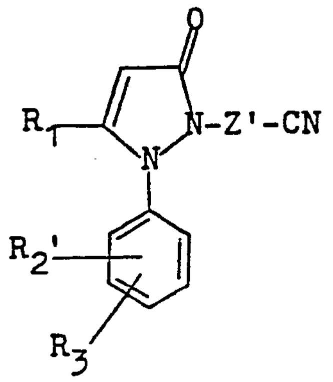

XII

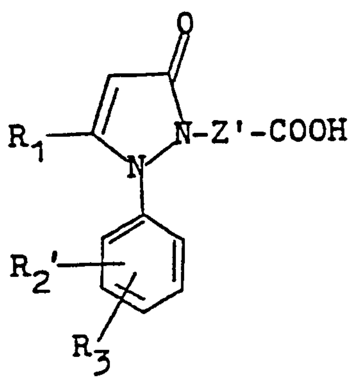

XIII

$H_2N-R_4$ XIV

**Patentansprüche für die Vertragsstaaten:**
BE, CH, DE, FR, GB, IT, LU, NL, SE

1. 5-Alkyl-1-phenyl-2-piperazinoalkylpyrazolin-3-on-Verbindungen der allgemeinen Formel I

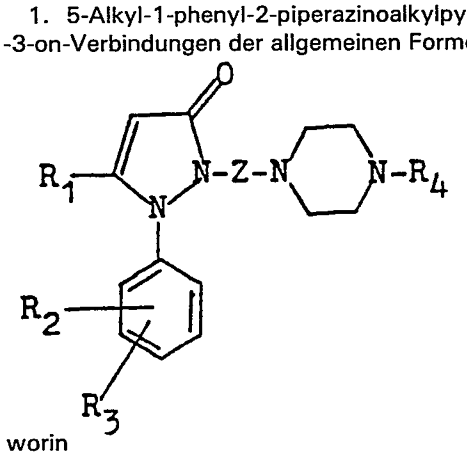

I

worin

$R_1$ eine geradkettige, verzweigte oder cyclische Alkylgruppe mit bis zu 6 Kohlenstoffatomen bedeutet,

$R_2$ Wasserstoff, Halogen, Nitro, Cyano, Hydroxy, Alkyl mit 1-4 Kohlenstoffatomen, Alkoxy mit 1-4 Kohlenstoffatomen, oder eine $R_5$-CO-Gruppe, worin $R_5$ für Alkoxy mit 1-4 Kohlenstoffatomen, Alkyl mit 1-4 Kohlenstoffatomen oder Hydroxy steht, bedeutet,

$R_3$ Wasserstoff, Halogen, Alkyl mit 1-4 Kohlenstoffatomen oder Alkoxy mit 1-4 Kohlenstoffatomen bedeutet,

Z eine Alkylenkette mit 2-4 Kohlenstoffatomen bedeutet,

$R_4$ eine Phenylgruppe der Formel a oder eine Pyridylgruppe der Formel b bedeutet,

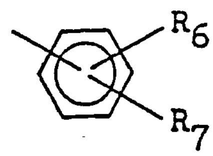

a)

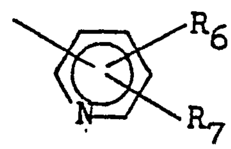

b)

worin

$R_6$ Wasserstoff, Halogen, Alkyl mit 1-4 Kohlenstoffatomen oder Alkoxy mit 1-4 Kohlenstoffatomen bedeutet, und

$R_7$ Wasserstoff, Halogen, Alkyl mit 1-4 Kohlenstoffatomen oder Alkoxy mit 1-4 Kohlenstoffatomen bedeutet,

sowie deren Säureadditionssalze.

2. 5-Alkyl-1-phenyl-2-piperazinoalkylpyrazolin-3-on-Verbindungen gemäß Anspruch 1, worin

$R_1$ und Z die in Anspruch 1 angegebene Bedeutung besitzen,

$R_2$ Wasserstoff, Halogen, Cyano, Nitro, Hydroxy, Alkyl mit 1-4 Kohlenstoffatomen, Alkoxy mit 1-4 Kohlenstoffatomen, Alkylcarbonyl mit 2-5 Kohlenstoffatomen oder Alkoxycarbonyl mit 2-5 Kohlenstoffatomen bedeutet,

$R_3$ Wasserstoff, Halogen oder Alkoxy mit 1-4 Kohlenstoffatomen bedeutet,

$R_4$ eine Phenylgruppe der Formel a bedeutet, worin

$R_6$ Wasserstoff, Halogen, Alkoxy mit 1-4 Kohlenstoffatomen oder Alkyl mit 1-4 Kohlenstoffatomen und

$R_7$ Wasserstoff, Halogen, Alkyl mit 1-4 Kohlenstoffatomen oder Alkoxy mit 1-4 Kohlenstoffatomen darstellen, oder

$R_4$ eine Pyridylgruppe der Formel b bedeutet, worin

$R_6$ Wasserstoff, Alkyl mit 1-4 Kohlenstoffatomen oder Halogen und

$R_7$ Wasserstoff darstellen.

3. 5-Alkyl-1-phenyl-2-piperazinoalkylpyrazolin-3-on-Verbindungen gemäß Anspruch 2, worin

$R_1$ obige Bedeutung besitzt,

Z eine Alkylenkette mit 3-4 Kohlenstoffatomen darstellt,

$R_2$ Wasserstoff, Halogen, Cyano, Nitro, Alkoxy mit 1-4 Kohlenstoffatomen, Alkylcarbonyl mit 2-5 Kohlenstoffatomen bedeutet,

$R_3$ Wasserstoff bedeutet, und

$R_4$ für eine Phenylgruppe steht, welche unsubstituiert oder durch Halogen substituiert ist, oder

$R_4$ für eine 2-Pyridylgruppe steht, welche unsubstituiert oder durch Alkyl mit 1-4 Kohlenstoffatomen substituiert ist.

4. 5-Alkyl-1-phenyl-2-piperazinoalkylpyrazolin-3-on-Verbindungen gemäß Anspruch 3, worin $R_1$ obige Bedeutung besitzt, $R_2$ Wasserstoff, Nitro, Chlor, Cyano, Methoxy oder Acetyl und $R_3$ Wasserstoff bedeuten, Z eine Propylenkette bedeutet und $R_4$ die 4-Methyl-2-pyridylgruppe oder die 4-Fluorphenylgruppe bedeutet.

5. 5-Alkyl-1-phenyl-2-piperazinoalkylpyrazolin-3-on-Verbindungen gemäß Anspruch 4, worin $R_2$, $R_3$, $R_4$ und Z die in Anspruch 4 angegebene Bedeutung besitzen und $R_1$ geradkettiges oder verzweigtes Alkyl mit 1-4 Kohlenstoffatomen oder cyclisches Alkyl mit 5 oder 6 Kohlenstoffatomen bedeutet.

6. 5-Alkyl-1-phenyl-2-(3-piperazin-1-ylpropyl)-pyrazolin-3-on-Verbindungen gemäß Anspruch 5, deren 1-Phenylring unsubstituiert oder in 4-Stellung durch Chlor, Nitro oder Methoxy substituiert ist, und deren 5-Alkylgruppe für Methyl, Äthyl, n-Propyl, Cyclopentyl oder Cyclohexyl steht.

7. 5-Alkyl-1-phenyl-2-(3-piperazin-1-ylpropyl)-pyrazolin-3-on-Verbindungen gemäß Anspruch 5, deren 5-Alkylgruppe $R_1$ für Cyclohexyl steht.

8. Verfahren zur Herstellung von 5-Alkyl-1-phenyl-2-piperazinoalkylpyrazolin-3-on-Verbindungen der allgemeinen Formel I

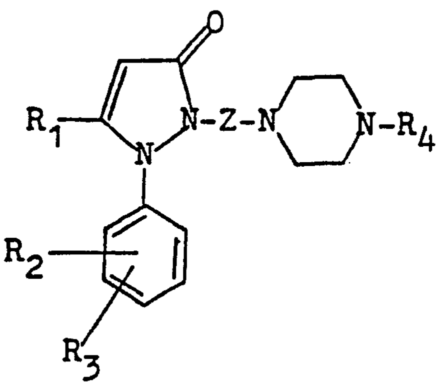

I

worin $R_1$, $R_2$, $R_3$, $R_4$ und Z die in Anspruch 1 angegebene Bedeutung besitzen,
sowie deren Säureadditionssalze, dadurch gekennzeichnet, daß man

a) Verbindungen der Formel II oder III

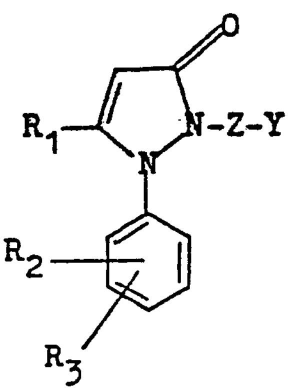

II

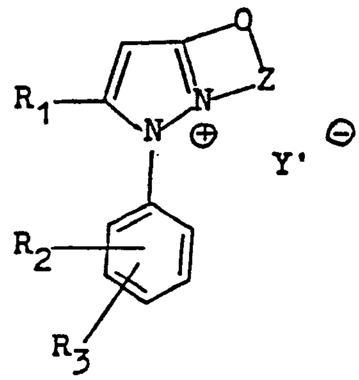

III

worin $R_1$, $R_2$, $R_3$ und Z obige Bedeutung besitzen, Y einen aminolytisch abspaltbaren Rest und Y' Halogen bedeuten, oder deren Gemische mit Verbindungen der Formel IV

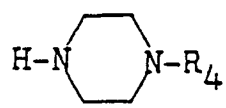

IV

worin $R_4$ obige Bedeutung besitzt, umsetzt oder

b) zur Herstellung von Verbindungen der allgemeinen Formel Ia

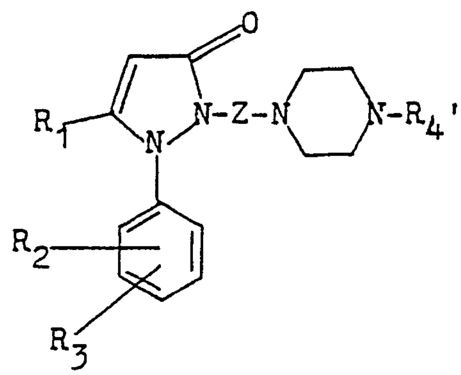

Ia

worin $R_1$, $R_2$, $R_3$ und Z obige Bedeutung besitzen und $R_4'$ für eine 2-Pyridylgruppe der Formel b'

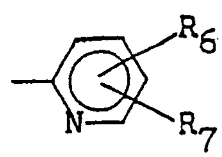

b'

worin $R_6$ und $R_7$ obige Bedeutung besitzen, steht, Verbindungen der Formel V

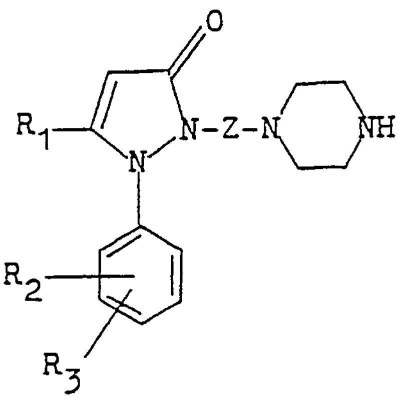

V

worin $R_1$, $R_2$, $R_3$ und Z obige Bedeutung besitzen, mit Verbindungen der Formel VI

Hal-$R_4'$ VI

worin $R_4'$ obige Bedeutung besitzt und Hal Halogen bedeutet, umsetzt, oder

c) zur Herstellung von Verbindungen der allgemeinen Formel Ib

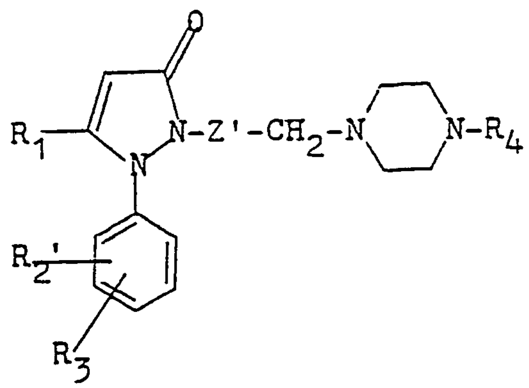

Ib

worin $R_1$, $R_3$ und $R_4$ obige Bedeutung besitzen, $R_2'$ die für $R_2$ angegebene Bedeutung mit Ausnahme von Cyano und von eine CO-Gruppe enthaltenden Resten besitzt und Z' eine Alkylenkette mit 2 oder 3 Kohlenstoffatomen bedeutet, Verbindungen der Formel VII

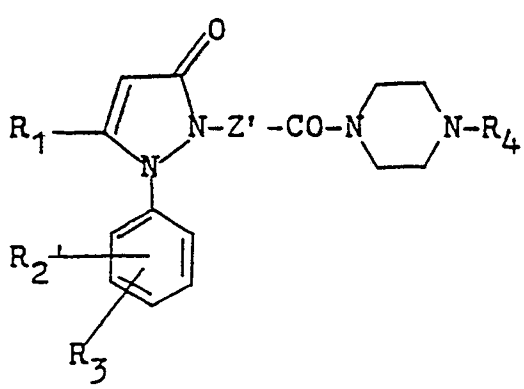

VII

worin $R_1$, $R_2'$, $R_3$, $R_4$ und Z' obige Bedeutung besitzen, reduziert und

gewünschtenfalls in erhaltenen Verbindungen der Formel I aus allfälligen Methoxysubstituenten die Hydroxygruppe oder aus allfälligen Alkoxycarbonylsubstituenten die Carboxylgruppe freisetzt und gegebenenfalls freie Verbindungen der Formel I in ihre Säureadditionssalze überführt oder die Säureadditionssalze in die freien Verbindungen der Formel I überführt.

9. Heilmittel, enthaltend eine pharmakologisch wirksame Menge einer 5-Alkyl-1-phenyl-2-piperazinoalkylpyrazolin-3-on-Verbindung gemäß Anspruch 1 und übliche pharmazeutische Hilfs- und/oder Trägerstoffe.

10. Verbindungen der allgemeinen Formel II oder III

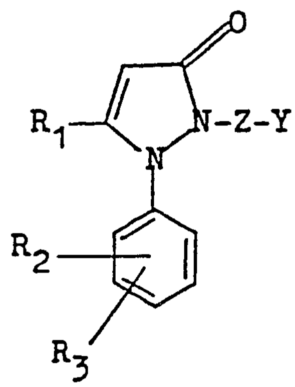

II

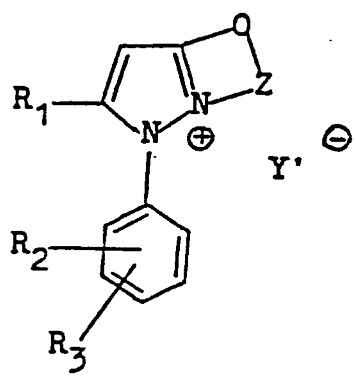

III

worin

$R_1$ eine geradkettige, verzweigte oder cyclische Alkylgruppe mit 2 bis 6 Kohlenstoffatomen bedeutet, oder fall $R_2$ und $R_3$ nicht beide Wasserstoff sind, auch Methyl bedeutet,

$R_2$ Wasserstoff, Halogen, Nitro, Cyano, Alkyl mit 1-4 Kohlenstoffatomen, Alkoxy mit 1-4 Kohlenstoffatomen oder eine $R_5$-CO-Gruppe, worin $R_5$ für Alkoxy mit 1-4 Kohlenstoffatomen oder Alkyl mit 1-4 Kohlenstoffatomen steht, bedeutet,

$R_3$ Wasserstoff, Halogen, Alkyl mit 1-4 Kohlenstoffatomen oder Alkoxy mit 1-4 Kohlenstoffatomen bedeutet,

Z eine Alkylenkette mit 2-4 Kohlenstoffatomen bedeutet, und

Y einen aminolytisch abspaltbaren Rest und Y' Halogen bedeuten.

11. Verbindungen der allgemeinen Formel V

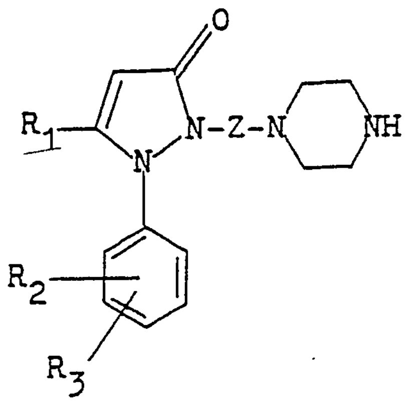

V

worin

$R_1$ eine geradkettige, verzweigte oder cyclische Alkylgruppe mit bis zu 6 Kohlenstoffatomen bedeutet,

$R_2$ Wasserstoff, Halogen, Nitro, Cyano, Alkyl mit 1-4 Kohlenstoffatomen, Alkoxy mit 1-4 Kohlenstoffatomen, oder eine $R_5$-CO-Gruppe, worin $R_5$ für Alkoxy mit 1-4 Kohlenstoffatomen, oder Alkyl mit 1-4 Kohlenstoffatomen steht, bedeutet,

$R_3$ Wasserstoff, Halogen, Alkyl mit 1-4 Kohlenstoffatomen oder Alkoxy mit 1-4 Kohlenstoffatomen bedeutet, und

Z eine Alkylenkette mit 2-4 Kohlenstoffatomen bedeutet.

12. Verbindungen der allgemeinen Formel VII

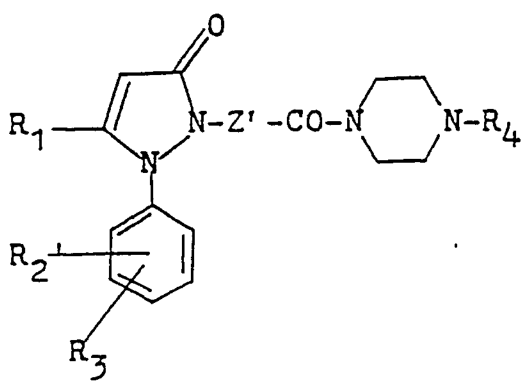

VII

worin

$R_1$ eine geradkettige, verzweigte oder cyclische Alkylgruppe mit bis zu 6 Kohlenstoffatomen bedeutet,

$R_2'$ Wasserstoff, Halogen, Nitro, Hydroxy, Alkyl mit 1-4 Kohlenstoffatomen oder Alkoxy mit 1-4 Kohlenstoffatomen bedeutet,

$R_3$ Wasserstoff, Halogen, Alkyl mit 1-4 Kohlenstoffatomen oder Alkoxy mit 1-4 Kohlenstoffatomen bedeutet,

Z' eine Alkylenkette mit 2 oder 3 Kohlenstoffatomen bedeutet,

$R_4$ eine Phenylgruppe der Formel a oder eine Pyridylgruppe der Formel b' bedeutet,

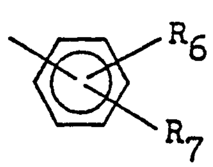

a)

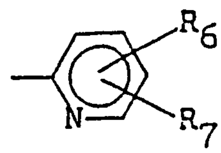

b'

worin

$R_6$ Wasserstoff, Halogen, Alkyl mit 1-4 Kohlenstoffatomen oder Alkoxy mit 1-4 Kohlenstoffatomen bedeutet, und

$R_7$ Wasserstoff, Halogen, Alkyl mit 1-4 Kohlenstoffatomen oder Alkoxy mit 1-4 Kohlenstoffatomen bedeutet,

sowie deren Säureadditionssalze.

**Patentansprüche für den Vertragsstaat:** AT

1. Verfahren zur Herstellung von 5-Alkyl-1-phenyl-2-piperazinoalkylpyrazolin-3-on-Verbindungen der allgemeinen Formel I

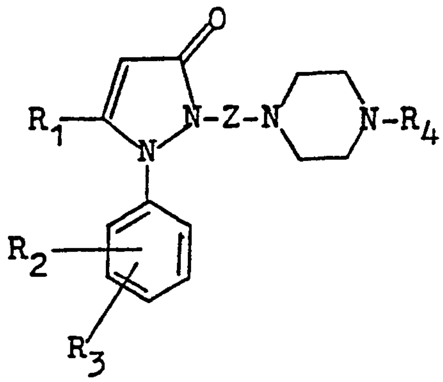

I

worin

$R_1$ eine geradkettige, verzweigte oder cyclische Alkylgruppe mit bis zu 6 Kohlenstoffatomen bedeutet,

$R_2$ Wasserstoff, Halogen, Nitro, Cyano, Hydroxy, Alkyl mit 1-4 Kohlenstoffatomen, Alkoxy mit 1-4 Kohlenstoffatomen, oder eine $R_5$-CO-Gruppe, worin $R_5$ für Alkoxy mit 1-4 Kohlenstoffatomen, Alkyl mit 1-4 Kohlenstoffatomen oder Hydroxy steht, bedeutet,

$R_3$ Wasserstoff, Halogen, Alkyl mit 1-4 Kohlenstoffatomen oder Alkoxy mit 1-4 Kohlenstoffatomen bedeutet,

Z eine Alkylenkette mit 2-4 Kohlenstoffatomen bedeutet,

$R_4$ eine Phenylgruppe der Formel a oder eine Pyridylgruppe der Formel b bedeutet,

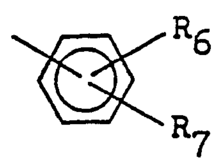

a)

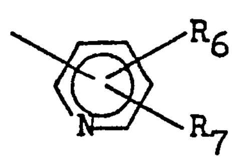

b)

worin

$R_6$ Wasserstoff, Halogen, Alkyl mit 1-4 Kohlenstoffatomen oder Alkoxy mit 1-4 Kohlenstoffatomen bedeutet, und

$R_7$ Wasserstoff, Halogen, Alkyl mit 1-4 Kohlenstoffatomen oder Alkoxy mit 1-4 Kohlenstoffatomen bedeutet,

sowie deren Säureadditionssalze, dadurch gekennzeichnet, daß man

a) Verbindungen der Formel II oder III

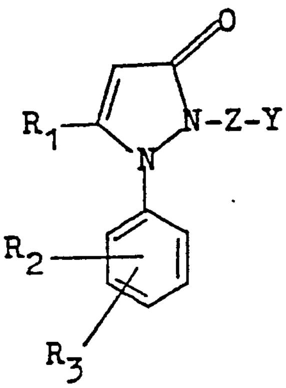

II

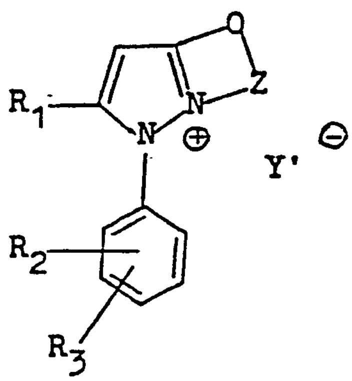

III

worin $R_1$, $R_2$, $R_3$ und Z obige Bedeutung besitzen, Y einen aminolytisch abspaltbaren Rest und Y' Halogen bedeuten, oder deren Gemische mit Verbindungen der Formel IV

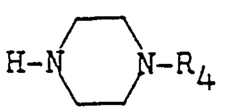

IV

worin $R_4$ obige Bedeutung besitzt, umsetzt oder

b) zur Herstellung von Verbindungen der allgemeinen Formel Ia

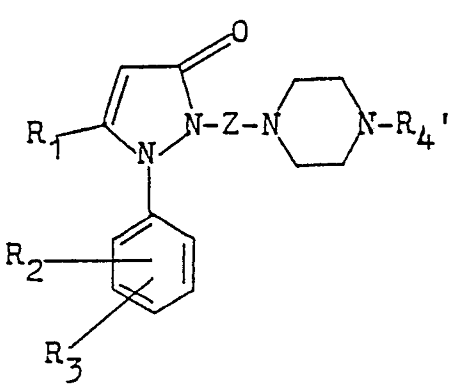

Ia

worin $R_1$, $R_2$, $R_3$ und Z obige Bedeutung besitzen und $R_4'$ für eine 2-Pyridylgruppe der Formel b'

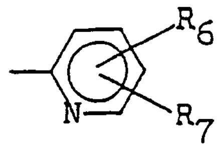

b'

worin $R_6$ und $R_7$ obige Bedeutung besitzen, steht, Verbindungen der Formel V

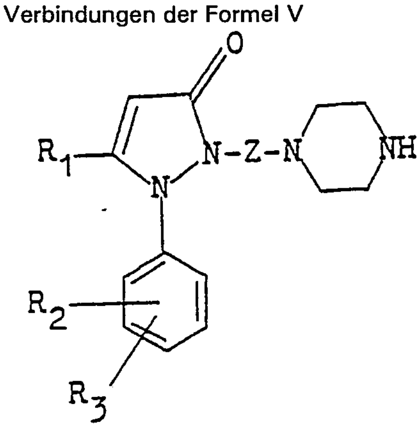

V

worin $R_1$, $R_2$, $R_3$ und Z obige Bedeutung besitzen, mit Verbindungen der Formel VI

Hal-$R_4'$ VI

worin $R_4'$ obige Bedeutung besitzt und Hal Halogen bedeutet, umsetzt, oder

c) zur Herstellung von Verbindungen der allgemeinen Formel Ib

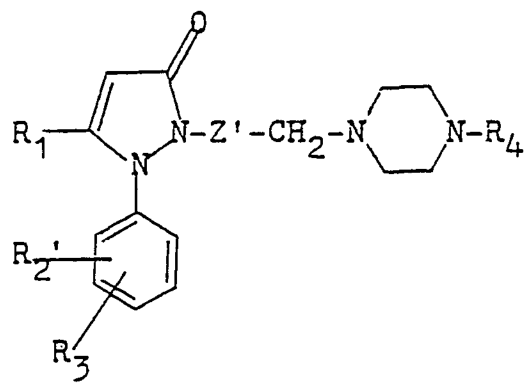

Ib

worin $R_1$, $R_3$ und $R_4$ obige Bedeutung besitzen, $R_2'$ die für $R_2$ angegebene Bedeutung mit Ausnahme von Cyano und von eine CO-Gruppe enthaltenden Resten besitzt und Z' eine Alkylenkette mit 2 oder 3 Kohlenstoffatomen bedeutet, Verbindungen der Formel VII

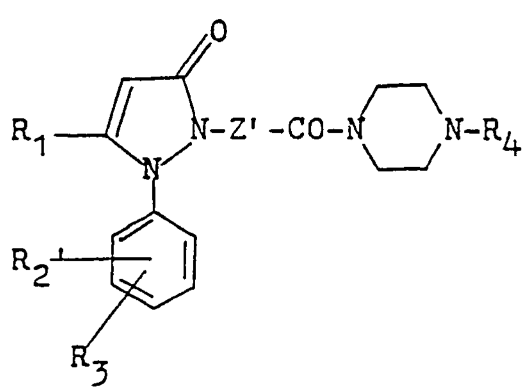

VII

worin $R_1$, $R_2'$, $R_3$, $R_4$ und Z' obige Bedeutung besitzen, reduziert und gewünschtenfalls in erhaltenen Verbindungen der Formel I aus allfälligen Methoxysubstituenten die Hydroxygruppe oder aus allfälligen Alkoxycarbonylsubstituenten die Carboxylgruppe freisetzt und gegebenenfalls freie Verbindungen der Formel I in ihre Säureadditionssalze überführt oder die Säureadditionssalze in die freien Verbindungen der Formel I überführt.

2. Verfahren nach Anspruch 1, dadurch gekennzeichnet, daß man als Verbindungen der Formel I 5-Alkyl-1-phenyl-2-piperazinoalkylpyrazolin-3-on--Verbindungen herstellt, worin

$R_1$ und Z die in Anspruch 1 angegebene Bedeutung besitzen,

$R_2$ Wasserstoff, Halogen, Cyano, Nitro, Hydroxy, Alkyl mit 1-4 Kohlenstoffatomen, Alkoxy mit 1-4 Kohlenstoffatomen, Alkylcarbonyl mit 2-5 Kohlenstoffatomen oder Alkoxycarbonyl mit 2-5 Kohlenstoffatomen bedeutet,

$R_3$ Wasserstoff, Halogen oder Alkoxy mit 1-4 Kohlenstoffatomen bedeutet,

$R_4$ eine Phenylgruppe der Formel a bedeutet, worin

$R_6$ Wasserstoff, Halogen, Alkoxy mit 1-4 Kohlenstoffatomen oder Alkyl mit 1-4 Kohlenstoffatomen und

$R_7$ Wasserstoff, Halogen, Alkyl mit 1-4 Kohlenstoffatomen oder Alkoxy mit 1-4 Kohlenstoffatomen darstellen, oder

$R_4$ eine Pyridylgruppe der Formel b bedeutet, worin

$R_6$ Wasserstoff, Alkyl mit 1-4 Kohlenstoffatomen oder Halogen und

$R_7$ Wasserstoff darstellen.

3. Verfahren nach Anspruch 2, dadurch gekennzeichnet, daß man als Verbindungen der Formel I 5-Alkyl-1-phenyl-2-piperazinoalkylpyrazolin-3-on--Verbindungen herstellt, worin

$R_1$ obige Bedeutung besitzt,

Z eine Alkylenkette mit 3-4 Kohlenstoffatomen darstellt,

$R_2$ Wasserstoff, Halogen, Cyano, Nitro, Alkoxy mit 1-4 Kohlenstoffatomen, Alkylcarbonyl mit 2-5 Kohlenstoffatomen bedeutet,

$R_3$ Wasserstoff bedeutet, und

$R_4$ für eine Phenylgruppe steht, welche unsubstituiert oder durch Halogen substituiert ist, oder

$R_4$ für eine 2-Pyridylgruppe steht, welche unsubstituiert oder durch Alkyl mit 1-4 Kohlenstoffatomen substituiert ist.

4. Verfahren nach Anspruch 3, dadurch gekennzeichnet, daß man als Verbindungen der Formel I 5-Alkyl-1-phenyl-2-piperazinoalkylpyrazolin-3-on--Verbindungen herstellt, worin $R_1$ obige Bedeutung besitzt, $R_2$ Wasserstoff, Nitro, Chlor, Cyano, Methoxy oder Acetyl und $R_3$ Wasserstoff bedeuten, Z eine Propylenkette bedeutet und $R_4$ die 4-Methyl-2--pyridylgruppe oder die 4-Fluorphenylgruppe bedeutet.

5. Verfahren nach Anspruch 4, dadurch gekennzeichnet, daß man als Verbindungen der Formel I 5-Alkyl-1-phenyl-2-piperazinoalkylpyrazolin-3-on--Verbindungen herstellt, worin $R_2$, $R_3$, $R_4$ und Z die in Anspruch 4 angegebene Bedeutung besitzen und $R_1$ geradkettiges oder verzweigtes Alkyl mit 1-4 Kohlenstoffatomen oder cyclisches Alkyl mit 5 oder 6 Kohlenstoffatomen bedeutet.

6. Verfahren nach Anspruch 5, dadurch gekennzeichnet, daß man als Verbindungen der Formel I 5-Alkyl-1-phenyl-2-(3-piperazin-1-ylpropyl)-pyrazolin-3-on-Verbindungen herstellt, deren 1-Phenylring unsubstituiert oder in 4-Stellung durch Chlor, Nitro oder Methoxy substituiert ist, und deren 5-Alkylgruppe für Methyl, Äthyl, n-Propyl, Cyclopentyl oder Cyclohexyl steht.

7. Verfahren nach Anspruch 6, dadurch gekennzeichnet, daß man als Verbindungen der Formel I 5-Alkyl-1-phenyl-2-(3-piperazin-1-ylpropyl)-pyrazolin-3-on-Verbindungen herstellt, deren 5-Alkylgruppe $R_1$ für Cyclohexyl steht.

8. Verfahren zur Herstellung von Verbindungen der allgemeinen Formel II oder III

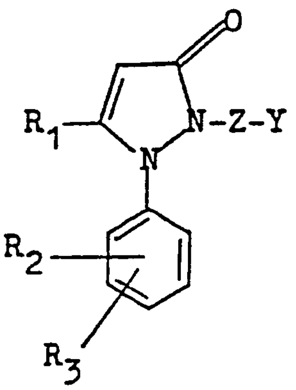

II

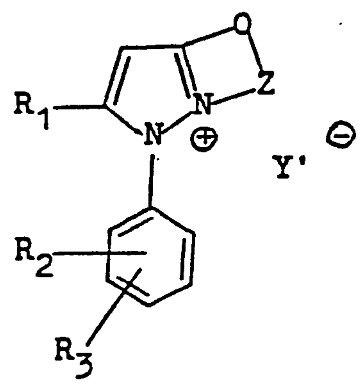

III

worin

$R_1$ eine geradkettige, verzweigte oder cyclische Alkylgruppe mit 2 bis 6 Kohlenstoffatomen bedeutet, oder falls $R_2$ und $R_3$ nicht beide Wasserstoff sind auch Methyl bedeutet,

$R_2$ Wasserstoff, Halogen, Nitro, Cyano, Alkyl mit 1-4 Kohlenstoffatomen, Alkoxy mit 1-4 Kohlenstoffatomen, oder eine $R_5$-CO-Gruppe, worin $R_5$ für Alkoxy mit 1-4 Kohlenstoffatomen, oder Alkyl mit 1-4 Kohlenstoffatomen steht, bedeutet,

$R_3$ Wasserstoff, Halogen, Alkyl mit 1-4 Kohlenstoffatomen oder Alkoxy mit 1-4 Kohlenstoffatomen bedeutet,

Z eine Alkylenkette mit 2-4 Kohlenstoffatomen bedeutet, und

Y einen aminolytisch abspaltbaren Rest und Y' Halogen bedeuten,
dadurch gekennzeichnet, daß man in situ hergestellte Alkalimetallsalze von Verbindungen der allgemeinen Formel VIII

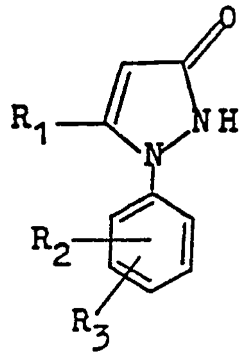

VIII

worin
$R_1$, $R_2$ und $R_3$ obige Bedeutung besitzen, mit Verbindungen der allgemeinen Formel IX

Y-Z-Y' IX

worin Z, Y und Y' obige Bedeutung besitzen, umsetzt und gegebenenfalls das erhaltene Gemisch von Verbindungen der Formel II und III auftrennt.

9. Verfahren zur Herstellung von Verbindungen der allgemeinen Formel V

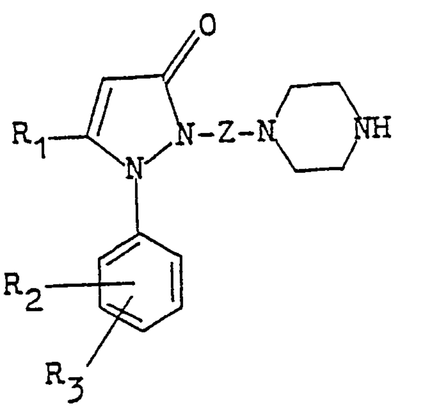

V

worin
Z eine Alkylenkette mit 2-4 Kohlenstoffatomen bedeutet,
$R_1$ eine geradkettige, verzweigte oder cyclische Alkylgruppe mit bis zu 6 Kohlenstoffatomen bedeutet,
$R_2$ Wasserstoff, Halogen, Nitro, Cyano, Alkyl mit 1-4 Kohlenstoffatomen, Alkoxy mit 1-4 Kohlenstoffatomen oder eine $R_5$-CO-Gruppe, worin $R_5$ für Alkoxy mit 1-4 Kohlenstoffatomen oder Alkyl mit 1-4 Kohlenstoffatomen steht, bedeutet, und
$R_3$ Wasserstoff, Halogen, Alkyl mit 1-4 Kohlenstoffatomen oder Alkoxy mit 1-4 Kohlenstoffatomen, bedeutet, dadurch gekennzeichnet, daß man
a) Verbindungen der allgemeinen Formeln II oder III

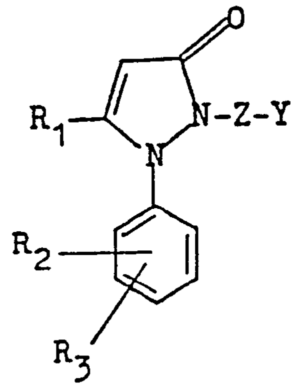

II

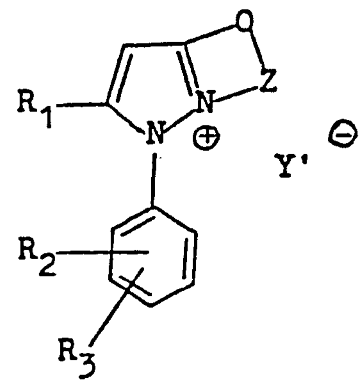

III

worin $R_1$, $R_2$, $R_3$ und Z obige Bedeutung besitzen, Y einen aminolytisch abspaltbaren Rest und Y' Halogen bedeuten, mit einem Überschuß Piperazin umsetzt, oder
b) aus Verbindungen der allgemeinen Formel X

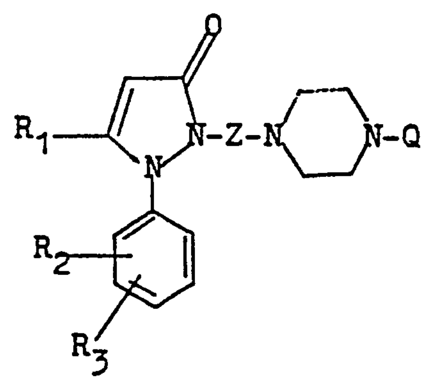

X

worin $R_1$, $R_2$, $R_3$ und Z obige Bedeutung besitzen und Q für eine Aminschutzgruppe steht, die Aminschutzgruppe abspaltet.

10. Verfahren zur Herstellung von Verbindungen der allgemeinen Formel VII

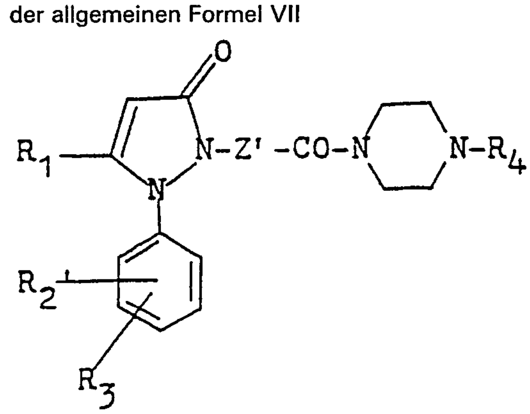

VII

worin

$R_1$ eine geradkettige, verzweigte oder cyclische Alkylgruppe mit bis zu 6 Kohlenstoffatomen bedeutet,

$R_2'$ Wasserstoff, Halogen, Nitro, Hydroxy, Alkyl mit 1-4 Kohlenstoffatomen oder Alkoxy mit 1-4 Kohlenstoffatomen, bedeutet,

$R_3$ Wasserstoff, Halogen, Alkyl mit 1-4 Kohlenstoffatomen oder Alkoxy mit 1-4 Kohlenstoffatomen bedeutet,

Z' eine Alkylenkette mit 2 oder 3 Kohlenstoffatomen bedeutet,

$R_4$ eine Phenylgruppe der Formel a oder eine Pyridylgruppe der Formel b' bedeutet,

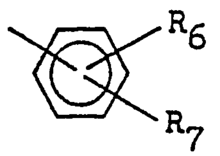

a)

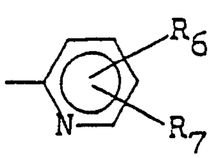

b'

worin

$R_6$ Wasserstoff, Halogen, Alkyl mit 1-4 Kohlenstoffatomen oder Alkoxy mit 1-4 Kohlenstoffatomen bedeutet, und

$R_7$ Wasserstoff, Halogen, Alkyl mit 1-4 Kohlenstoffatomen oder Alkoxy mit 1-4 Kohlenstoffatomen bedeutet,

sowie deren Säureadditionssalze, dadurch gekennzeichnet, daß man Verbindungen der allgemeinen Formel XIII

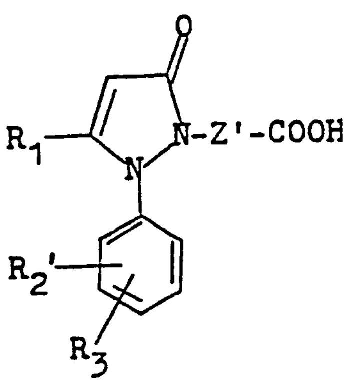

XIII

worin $R_1$, $R_2$, $R_3$ und Z' obige Bedeutung besitzen, mit Verbindungen der allgemeinen Formel IV

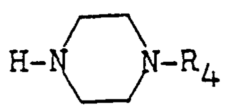

IV

worin $R_4$ obige Bedeutung besitzt, umsetzt und gegebenenfalls freie Verbindungen der Formel VII in Säureadditionssalze überführt oder die Säureadditionssalze in die freien Verbindungen der Formel VII überführt.

**Claims for the Contracting States:**
BE, CH, DE, FR, GB, IT, LU, NL, SE

1. 5-alkyl-1-phenyl-2-piperazinoalkylpyrazolin-3-one compounds of the general formula I,

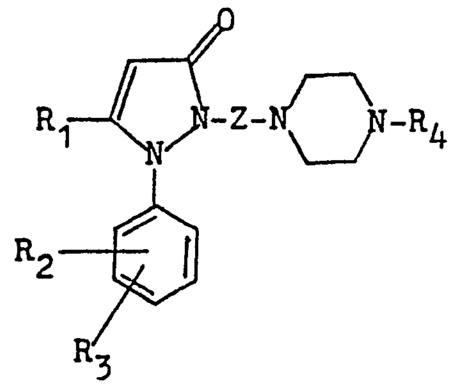

I

wherein

$R_1$ is a straight-chain, branched or cyclic alkyl group with up to 6 carbon atoms,

$R_2$ is hydrogen, halogen, nitro, cyano, hydroxy, alkyl with 1-4 carbon atoms, alkoxy with 1-4 carbon atoms or an $R_5$-CO-group, wherein $R_5$ stands for alkoxy with 1-4 carbon atoms, alkyl with 1-4 carbon atoms or hydroxy,

$R_3$ is hydrogen, halogen, alkyl with 1-4 carbon atoms or alkoxy with 1-4 carbon atoms,

Z is an alkylene chain with 2-4 carbon atoms,

$R_4$ is a phenyl group of formula a or a pyridyl group of formula b,

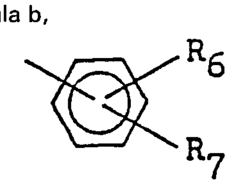

a)

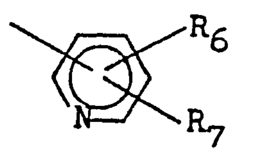

b)

wherein

$R_6$ is hydrogen, halogen, alkyl with 1-4 carbon atoms or alkoxy with 1-4 carbon atoms, and

$R_7$ is hydrogen, halogen alkyl with 1-4 carbon atoms or alkoxy with 1-4 carbon atoms,

and their acid addition salts.

2. 5-alkyl-1-phenyl-2-piperazinoalkylpyrazolin-3-one compounds according to Claim 1, wherein

$R_1$ and Z have the meanings given in Claim 1,

$R_2$ is hydrogen, halogen, cyano, nitro, hydroxy, alkyl with 1-4 carbon atoms, alkoxy with 1-4 carbon atoms, alkylcarbonyl with 2-5 carbon atoms or alkoxycarbonyl with 2-5 carbon atoms,

$R_3$ is hydrogen, halogen or alkoxy with 1-4 carbon atoms,

$R_4$ is a phenyl group of formula a, wherein

$R_6$ is hydrogen, halogen alkoxy with 1-4 carbon atoms or alkyl with 1-4 carbon atoms and

$R_7$ is hydrogen, halogen, alkyl with 1-4 carbon atoms or alkoxy with 1-4 carbon atoms, or

$R_4$ is a pyridyl group of formula b, wherein

$R_6$ is hydrogen, alkyl with 1-4 carbon atoms or halogen, and

$R_7$ is hydrogen.

3. 5-alkyl-1-phenyl-2-piperazinoalkylpyrazolin-3--one compounds according to Claim 2, wherein

$R_1$ has the above meaning,

Z represents an alkylene chain with 3-4 carbon atoms,

$R_2$ is hydrogen, halogen, cyano, nitro, alkoxy with 1-4 carbon atoms, alkylcarbonyl with 2-5 carbon atoms,

$R_3$ is hydrogen, and

$R_4$ stands for a phenyl group which is un-substituted or is substituted by halogen, or

$R_4$ stands for a 2-pyridyl group which is un-substituted or is substituted by alkyl with 1-4 carbon atoms.

4. 5-alkyl-1-phenyl-2-piperazinoalkylpyrazolin-3--one compounds according to Claim 3, wherein $R_1$ has the above meaning, $R_2$ is hydrogen, nitro, chlorine, cyano, methoxy or acetyl and $R_3$ is hydrogen, Z is a propylene chain and $R_4$ is the 4-methyl-2-pyridyl group or the 4-fluorophenyl group.

5. 5-alkyl-1-phenyl-2-piperazinoalkylpyrazolin-3--one compounds according to Claim 4, wherein $R_2$, $R_3$, $R_4$ and Z have the meanings given in Claim 4 and $R_1$ is straight-chain or branched alkyl with 1-4 carbon atoms or cyclic alkyl with 5 or 6 carbon atoms.

6. 5-alkyl-1-phenyl-2-(3-piperazin-1-ylpropyl)--pyrazolin-3-one compounds according to Claim 5, the 1-phenyl ring of which is un-substituted or is substituted in the 4-position by chlorine, nitro or methoxy, and the 5-alkyl group of which stands for methyl, ethyl, n-propyl, cyclopentyl or cyclohexyl.

7. 5-alkyl-1-phenyl-2-(3-piperazin-1-ylpropyl)--pyrazolin-3-one compounds according to Claim 5, the 5-alkyl group $R_1$ of which stands for cyclohexyl.

8. A process for producing 5-alkyl-1-phenyl-2--piperazinoalkylpyrazolin-3-one compounds of the general formula I,

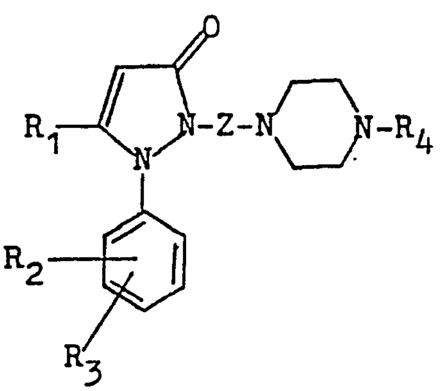

I

wherein $R_1$, $R_2$, $R_3$, $R_4$ and Z have the meanings given in Claim 1,

and their acid addition salts, characterised in that

a) compounds of formula II or III,

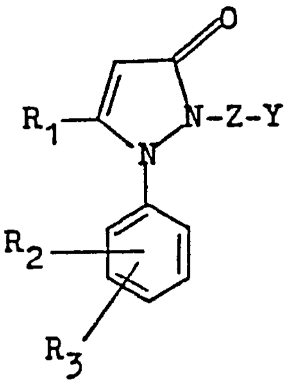

II

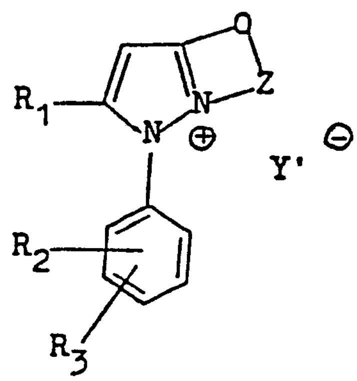

III

wherein $R_1$, $R_2$, $R_3$ and Z have the above meanings, Y is an aminolytically cleavable radical and Y' is halogen, or mixtures thereof are reacted with compounds of formula IV,

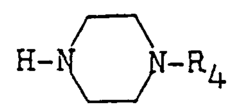

IV

wherein $R_4$ has the above meaning, or

b) to produce compounds of the general formula Ia,

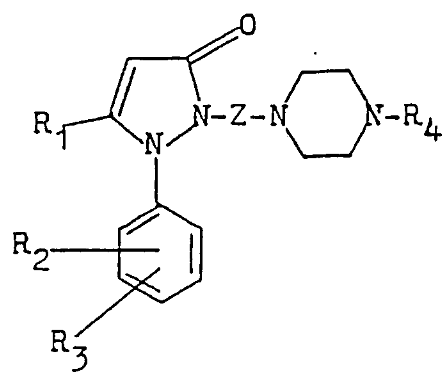

Ia

wherein $R_1$, $R_2$, $R_3$ and Z have the above meanings and $R_4'$ stands for a 2-pyridyl group of formula b',

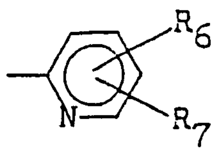

b'

wherein $R_6$ and $R_7$ have the above meanings, compounds of formula V,

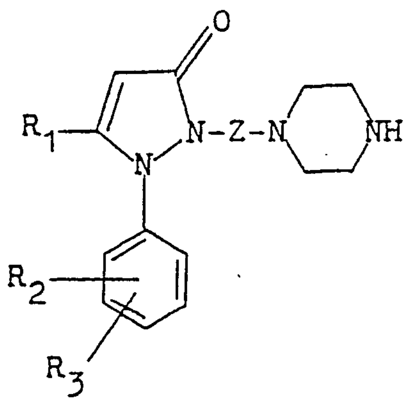

V

wherein $R_1$, $R_2$, $R_3$ and Z have the above meanings, are reacted with compounds of formula VI,

Hal-$R_4'$ VI

wherein $R_4'$ has the above meaning and Hal stands for halogen, or

c) to produce compounds of the general formula Ib,

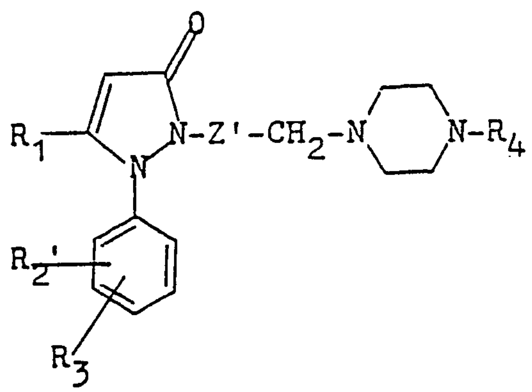

Ib

wherein $R_1$, $R_3$ and $R_4$ have the above meanings, $R_2'$ has the meaning given for $R_2$ with the exception of cyano and of radicals containing a CO-group, and Z' is an alkylene chain with 2 or 3 carbon atoms, compounds of formula VII,

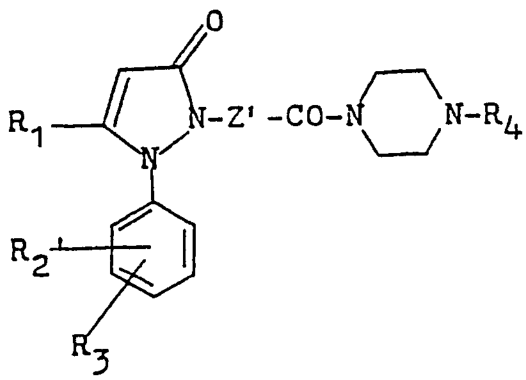

VII

wherein $R_1$, $R_2'$, $R_3$, $R_4$ and Z' have the above meanings, are reduced and

if desired, in resulting compounds of formula I the hydroxy group is released from any methoxy substituents or the carboxyl group is released from any alkoxycarbonyl substituents and optionally free compounds of formula I are converted into their acid addition salts or the acid addition salts are converted into the free compounds of formula I.

9. Medicament, containing a pharmacologically effective quantity of a 5-alkyl-1-phenyl-2-piperazinoalkylpyrazolin-3-one compound according to Claim 1 and conventional pharmaceutical auxiliaries and/or carriers.

10. Compounds of the general formula II or III,

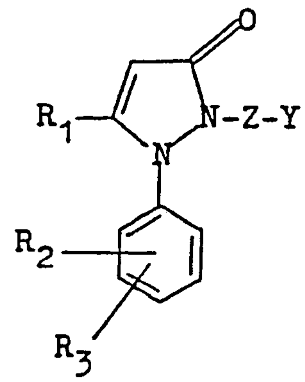

II

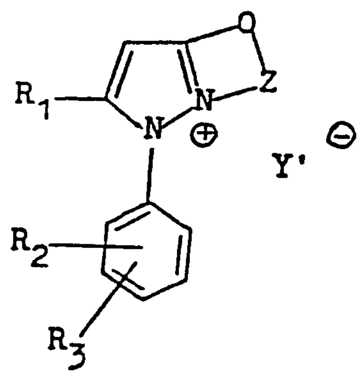

III

wherein

$R_1$ is a straight-chain, branched or cyclic alkyl group with 2 to 6 carbon atoms, or, if $R_2$ and $R_3$ are not both hydrogen, is also methyl,

$R_2$ is hydrogen, halogen, nitro, cyano, alkyl with 1-4 carbon atoms or an $R_5$-CO-group, wherein $R_5$ stands for alkoxy with 1-4 carbon atoms or alkyl with 1-4 carbon atoms,

$R_3$ is hydrogen, halogen, alkyl with 1-4 carbon atoms or alkoxy with 1-4 carbon atoms,

Z is an alkylene chain with 2-4 carbon atoms, and

Y is an aminolytically cleavable radical and Y' is halogen.

11. Compounds of the general formula V,

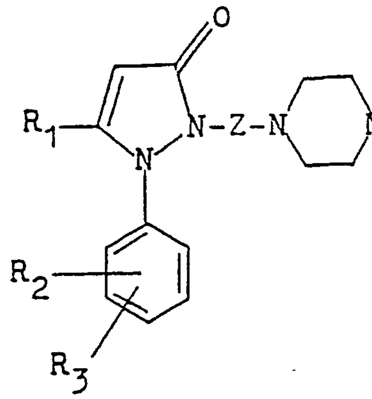

V

wherein

$R_1$ is a straight-chain, branched or cyclic alkyl group with up to 6 carbon atoms,

$R_2$ is hydrogen, halogen, nitro, cyano, alkyl with 1-4 carbon atoms, alkoxy with 1-4 carbon atoms or an $R_5$-CO-group, wherein $R_5$ stands for alkoxy with 1-4 carbon atoms or alkyl with 1-4 carbon atoms,

$R_3$ is hydrogen, halogen, alkyl with 1-4 carbon atoms or alkoxy with 1-4 carbon atoms, and

Z is an alkylene chain with 2-4 carbon atoms.

12. Compounds of the general formula VII,

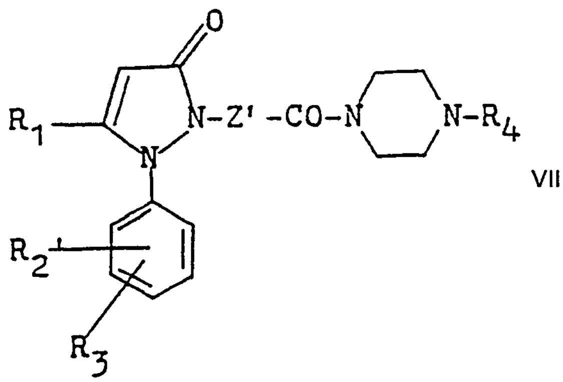

VII

wherein

$R_1$ is a straight-chain, branched or cyclic alkyl group with up to 6 carbon atoms,

$R_2'$ is hydrogen, halogen, nitro, hydroxy, alkyl with 1-4 carbon atoms or alkoxy with 1-4 carbon atoms,

$R_3$ is hydrogen, halogen, alkyl with 1-4 carbon atoms or alkoxy with 1-4 carbon atoms,

Z' is an alkylene chain with 2 or 3 carbon atoms,

$R_4$ is a phenyl group of formula a or a pyridyl group of formula b'

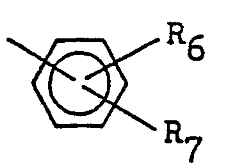

a)

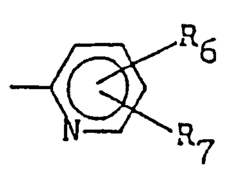

b'

wherein

$R_6$ is hydrogen, halogen, alkyl with 1-4 carbon atoms or alkoxy with 1-4 carbon atoms, and

$R_7$ hydrogen, halogen, alkyl with 1-4 carbon atoms or alkoxy with 1-4 carbon atoms,
and their acid addition salts.

**Claims for the Contracting State:** AT

1. A process for producing 5-alkyl-1-phenyl-2--piperazinoalkylpyrazolin-3-one compounds of the general formula I,

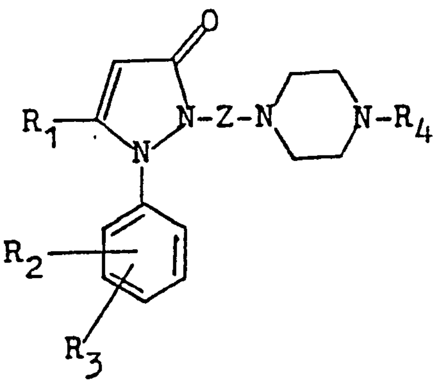

I

wherein

$R_1$ is a straight-chain, branched or cyclic alkyl group with up to 6 carbon atoms,

$R_2$ is hydrogen, halogen, nitro, cyano, hydroxy, alkyl with 1-4 carbon atoms, alkoxy with 1-4 carbon atoms or an $R_5$-CO-group, wherein $R_5$ stands for alkoxy with 1-4 carbon atoms, alkyl with 1-4 carbon atoms or hydroxy,

$R_3$ is hydrogen, halogen, alkyl with 1-4 carbon atoms or alkoxy with 1-4 carbon atoms,

Z is an alkylene chain with 2-4 carbon atoms,

$R_4$ is a phenyl group of formula a or a pyridyl group of formula b,

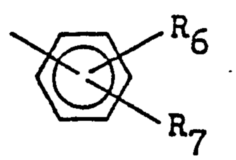

a)

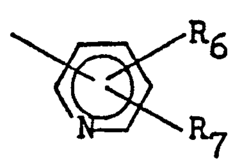

b)

wherein

$R_6$ is hydrogen, halogen, alkyl with 1-4 carbon atoms or alkoxy with 1-4 carbon atoms, and

$R_7$ is hydrogen, halogen, alkyl with 1-4 carbon atoms or alkoxy with 1-4 carbon atoms,
and their acid addition salts, characterised in that

a) compounds of formula II or III,

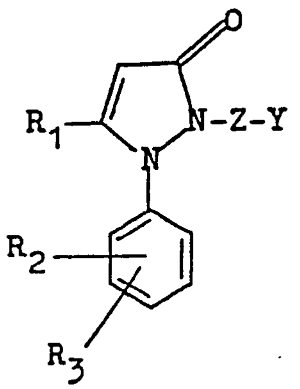

II

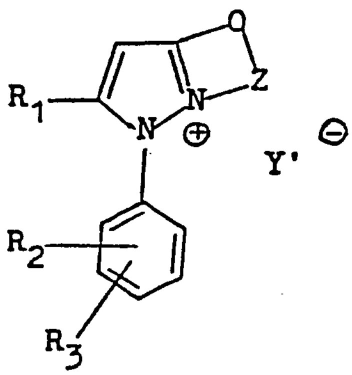

III

wherein $R_1$, $R_2$, $R_3$ and Z have the above meanings, Y is an aminolytically cleavable radical and Y' is halogen, or mixtures thereof are reacted with compounds of formula IV,

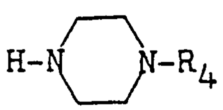

IV

wherein $R_4$ has the abover meaning, or

b) to produce compounds of the general formula Ia,

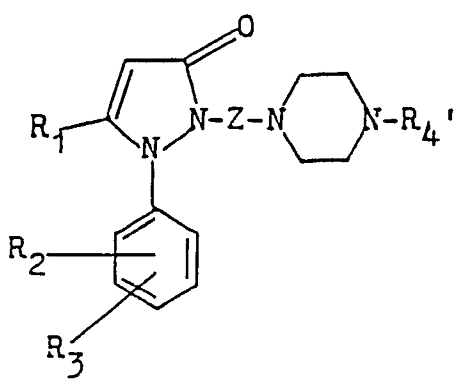

Ia

wherein $R_1$, $R_2$, $R_3$ and Z have the above meanings and $R_4'$ stands for a pyridyl group of formula b',

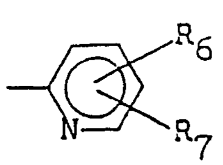

b'

wherein $R_6$ and $R_7$ have the above meanings, compounds of formula V,

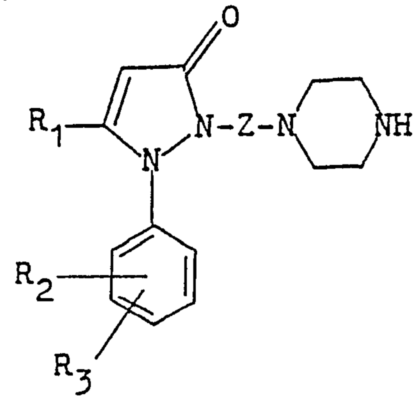

V

wherein $R_1$, $R_2$, $R_3$ and Z have the above meanings, are reacted with compounds of formula VI,

Hal-$R_4'$ VI

wherein $R_4'$ has the above meaning and Hal stands for halogen, or

c) to produce compounds of the general formula Ib,

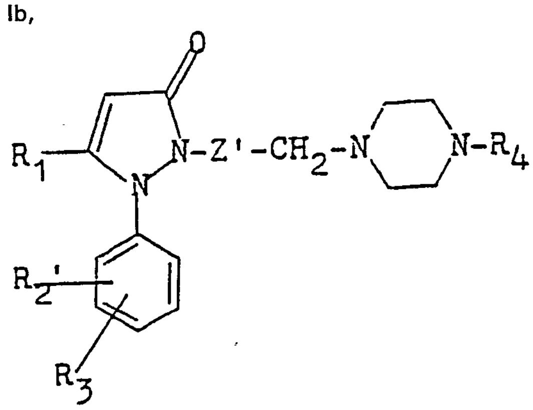

Ib

wherein $R_1$, $R_3$ and $R_4$ have the above meanings, $R_2'$ has the meaning given for $R_2$ with the exception of cyano and of radicals containing a CO-group, and Z' is an alkylene chain with 2 or 3 carbon atoms, compounds of formula VII,

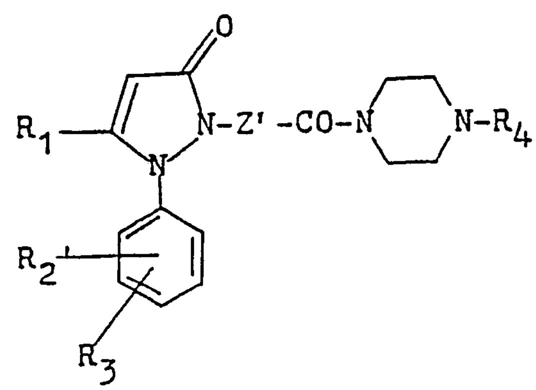

VII

wherein $R_1$, $R_2'$, $R_3$, $R_4$ and Z' have the above meanings, are reduced and

if desired, in resulting compounds of formula I the hydroxy group is released from any methoxy substituents or the carboxyl group is released from any alkoxycarbonyl substituents and optionally free

compounds of formula I are converted into their acid addition salts or the acid addition salts are converted into the free compounds of formula I.

2. A process according to Claim 1, characterised in that 5-alkyl-1-phenyl-2-piperazinoalkylpyrazolin-3-one compounds are produced as compounds of formula I, wherein

$R_1$ and Z have the meanings given in Claim 1,

$R_2$ is hydrogen, halogen, cyano, nitro, hydroxy, alkyl with 1-4 carbon atoms, alkoxy with 1-4 carbon atoms, alkylcarbonyl with 2-5 carbon atoms or alkoxycarbonyl with 2-5 carbon atoms,

$R_3$ is hydrogen, halogen or alkoxy with 1-4 carbon atoms,

$R_4$ is a phenyl group of formula a, wherein

$R_6$ is hydrogen, halogen, alkoxy with 1-4 carbon atoms or alkyl with 1-4 carbon atoms and

$R_7$ is hydrogen, halogen, alkyl with 1-4 carbon atoms or alkoxy with 1-4 carbon atoms, or

$R_4$ is a pyridyl group of formula b, wherein

$R_6$ is hydrogen, alkyl with 1-4 carbon atoms or halogen, and

$R_7$ is hydrogen.

3. A process according to Claim 2, characterised in that 5-alkyl-1-phenyl-2-piperazinoalkylpyrazolin-3-one compounds are produced as compounds of formula I, wherein

$R_1$ has the above meaning,

Z represents an alkylene chain with 3-4 carbon atoms,

$R_2$ is hydrogen, halogen, cyano, nitro, alkoxy with 1-4 carbon atoms, alkylcarbonyl with 2-5 carbon atoms,

$R_3$ is hydrogen, and

$R_4$ stands for a phenyl group which is un-substituted or is substituted by halogen, or

$R_4$ stands for a 2-pyridyyl group which is un-substituted or is substituted by alkyl with 1-4 carbon atoms.

4. A process according to Claim 3, characterised in that 5-alkyl-1-phenyl-2-piperazinoalkylpyrazolin-3-one compounds are produced as compounds of formula I, wherein $R_1$ has the above meaning, $R_2$ is hydrogen, nitro, chlorine, cyano, methoxy or acetyl and $R_3$ is hydrogen, Z is a propylene chain and $R_4$ is the 4-methyl-2-pyridyl group or the 4-fluorophenyl group.

5. A process according to Claim 4, characterised in that 5-alkyl-1-phenyl-2-piperazinoalkylpyrazolin-3-one compounds are produced as compounds of formula I, wherein $R_2$, $R_3$, $R_4$ and Z have the meanings given in Claim 4 and $R_1$ is straight-chain or branched alkyl with 1-4 carbon atoms or cyclic alkyl with 5 or 6 carbon atoms.

6. A process according to Claim 5, characterised in that 5-alkyl-1-phenyl-2-(3-piperazin-1-ylpropyl)-pyrazolin-3-one compounds are produced as compounds of formula I, the 1-phenyl ring of which is unsubstituted or is substituted in the 4-position by chlorine, nitro or methoxy, and the 5-alkyl group of which stands for methyl, ethyl, n-propyl, cyclopentyl or cyclohexyl.

7. A process according to Claim 6, characterised in that 5-alkyl-1-phenyl-2-(3-piperazin-1-ylpropyl)-pyrazolin-3-one compounds are produced as compounds of formula I, the 5-alkyl group $R_1$ of which stands for cyclohexyl.

8. A process for producing compounds of the general formula II or III,

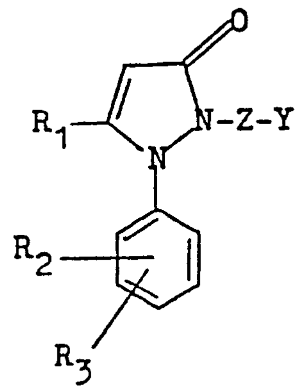

II

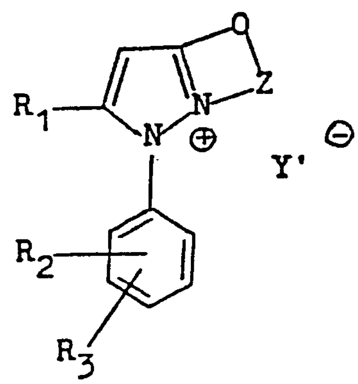

III

wherein

$R_1$ is a straight-chain, branched or cyclic alkyl group with 2 to 6 carbon atoms, or, if $R_2$ and $R_3$ are not both hydrogen, is also methyl,

$R_2$ is hydrogen, halogen, nitro, cyano, alkyl with 1-4 carbon atoms, alkoxy with 1-4 carbon atoms or an $R_5$-CO-group, wherein $R_5$ stands for alkoxy with 1-4 carbon atoms or alkyl with 1-4 carbon atoms,

$R_3$ is hydrogen, halogen, alkyl with 1-4 carbon atoms or alkoxy with 1-4 carbon atoms,

Z is an alkylene chain with 2-4 carbon atoms, and

Y is an aminolytically cleavable radical and Y' is halogen,

characterised in that alkali metal salts, produced in situ, of compounds of the general formula VIII,

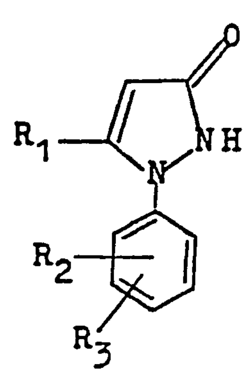

VIII

wherein

$R_1$, $R_2$ and $R_3$ have the above meanings, are reacted with compounds of the general formula IX,

Y-Z-Y' IX

wherein Z, Y and Y' have the above meanings, and optionally the resulting mixture of compounds of formula II and III is separated.

9. A process for producing compounds of the general formula V,

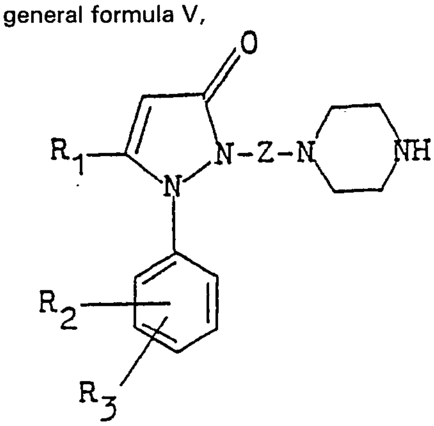

V

wherein

Z is an alkylene chain with 2-4 carbon atoms,

$R_1$ is a straight-chain, branched or cyclic alkyl group with up to 6 carbon atoms,

$R_2$ is hydrogen, halogen, nitro, cyano, alkyl with 1-4 carbon atoms, alkoxy with 1-4 carbon atoms or an $R_5$-CO-group, wherein $R_5$ stands for alkoxy with 1-4 carbon atoms or alkyl with 1-4 carbon atoms, and

$R_3$ is hydrogen, halogen, alkyl with 1-4 carbon atoms or alkoxy with 1-4 carbon atoms, characterised in that

a) compounds of the general formula II or III,

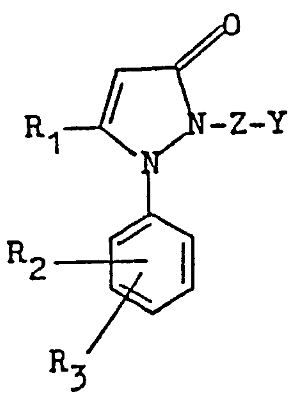

II

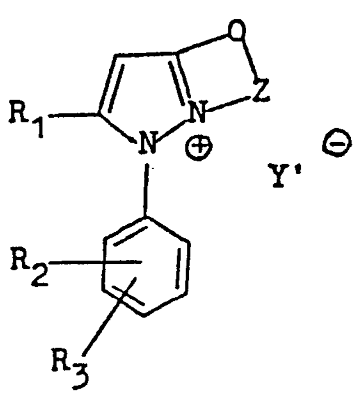

III

wherein $R_1$, $R_2$, $R_3$ and Z have the above meanings, Y is an aminolytically cleavable radical and Y' is halogen, are reacted with an excess of piperazine, or

b) the amino protecting group is split off from compounds of the general formula X,

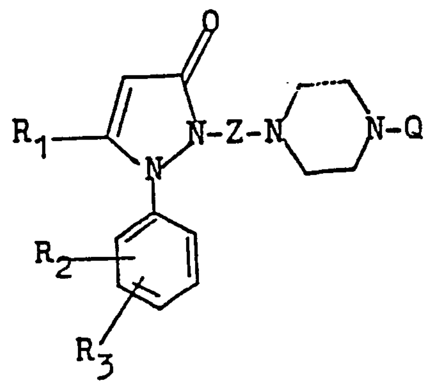

X

wherein $R_1$, $R_2$, $R_3$ and Z have the above meanings and Q stands for an amino protecting group.

10. A process for producing compounds of the general formula VII,

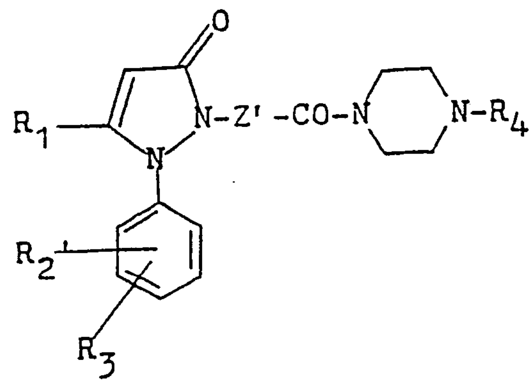

VII

wherein

$R_1$ is a straight-chain, branched or cyclic alkyl group with up to 6 carbon atoms,

$R_2'$ is hydrogen, halogen, nitro, hydroxy, alkyl with 1-4 carbon atoms or alkoxy with 1-4 carbon atoms,

$R_3$ is hydrogen, halogen, alkyl with 1-4 carbon atoms or alkoxy with 1-4 carbon atoms,

Z' is an alkylene chain with 2 or 3 carbon atoms,

$R_4$ is a phenyl group of formula a or a pyridyl group of formula b',

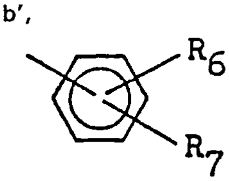

a)

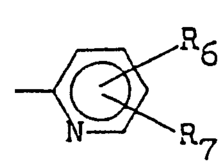

b'.

wherein

$R_6$ is hydrogen, halogen, alkyl with 1-4 carbon atoms or alkoxy with 1-4 carbon atoms, and

$R_7$ hydrogen, halogen, alkyl with 1-4 carbon atoms or alkoxy with 1-4 carbon atoms,
and their acid addition salts, characterised in that compounds of the general formula XIII,

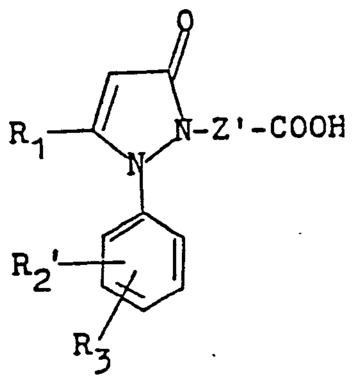

XIII

wherein $R_1$, $R_2$, $R_3$ and Z' have the above meanings, are reacted with compounds of the general formula IV,

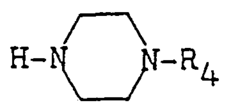

IV

wherein $R_4$ has the above meaning, and optionally free compounds of formula VII are converted into acid addition salts or the acid addition salts are converted into the free compounds of formula VII.

**Revendications pour les Etats contractants:**
BE, CH, DE, FR, GB, IT, LU, NL, SE

1. Composés de 5-alkyl-1-phényl-2-pipérazinoalkyl-pyrazoline-3-one de formule générale I

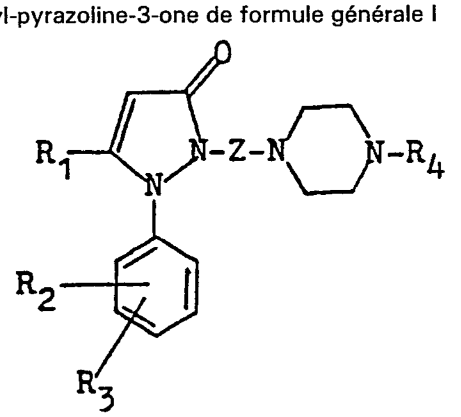

I

dans laquelle

$R_1$ est un groupe alkyle à chaîne droite, ramifiée ou cyclique, ayant jusqu'à 6 atomes de carbone,

$R_2$ est un hydrogène, un halogène, le radical nitro, cyano, hydroxy, un radical alkyle ayant de 1 à 4 atomes de carbone, alcoxy ayant de 1 à 4 atomes de carbone, ou un groupe $R_5$-CO dans lequel $R_5$ représente un radical alcoxy ayant de 1 à 4 atomes de carbone, alkyle ayant de 1 à 4 atomes de carbone ou hydroxy,

$R_3$ est un hydrogène ou un halogène, un alkyle ayant de 1 à 4 atomes de carbone ou un alcoxy ayant de 1 à 4 atomes de carbone,

Z est un enchaînement alkylène ayant de 2 à 4 atomes de carbone,

$R_4$ est un groupe phényle de formule a ou un groupe pyridyle de formule b

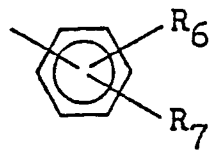

a)

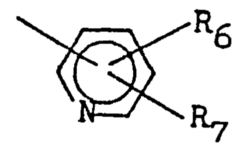

b)

où

$R_6$ est un hydrogène ou un halogène, un radical alkyle ayant de 1 à 4 atomes de carbone ou alcoxy ayant de 1 à 4 atomes de carbone, et

$R_7$ est un hydrogène ou un halogène, un radical alkyle ayant de 1 à 4 atomes de carbone ou alcoxy ayant de 1 à 4 atomes de carbone, ainsi que leurs sels d'addition avec un acide.

2. Composés de 5-alkyl-1-phényl-2-pipérazinoalkyl-pyrazoline-3-one selon la revendication 1, dans lesquels

$R_1$ et Z ont les significations données dans la revendication 1,

$R_2$ est un hydrogène, un halogène, le radical cyano, nitro, hydroxy, ou un radical alkyle ayant de 1 à 4 atomes de carbone, alcoxy ayant de 1 à 4 atomes de carbone, alkylcarbonyle ayant de 2 à 5 atomes de carbone ou alcoxycarbonyle ayant de 2 à 5 atomes de carbone,

$R_3$ est un hydrogène ou un halogène ou un alcoxy ayant de 1 à 4 atomes de carbone,

$R_4$ est un groupe phényle de formule a, dans laquelle

$R_6$ est un hydrogène ou un halogène, un alcoxy ayant de 1 à 4 atomes de carbone ou un alkyle ayant de 1 à 4 atomes de carbone, et

$R_7$ est un hydrogène ou un halogène, un alkyle ayant de 1 à 4 atomes de carbone ou un alcoxy ayant de 1 à 4 atomes de carbone ou bien

$R_4$ est un groupe pyridyle de formule b, dans laquelle

$R_6$ est un hydrogène ou un radical alkyle ayant de 1 à 4 atomes de carbone ou un halogène, et

$R_7$ est un hydrogène.

3. Composés de 5-alkyl-1-phényl-2-pipérazinoalkyl-pyrazoline-3-one selon la revendication 2, dans lesquels

$R_1$ a la signification ci-dessus,

Z est un enchaînement alkylène ayant 3-4 atomes de carbone,

$R_2$ est un hydrogène, un halogène, le radical cyano, nitro, un radical alcoxy ayant de 1 à 4 atomes de carbone ou alkylcarbonyle ayant de 2 à 5 atomes de carbone,

$R_3$ est un hydrogène, et

$R_4$ est un groupe phényle qui est non-substitué ou est substitué par un halogène, ou bien

$R_4$ est un groupe 2-pyridyle qui est non-substitué ou est substitué par un radical alkyle ayant de 1 à 4 atomes de carbone.

4. Composés de 5-alkyl-1-phényl-2-pipérazinoalkyl-pyrazoline-3-one selon la revendication 3, dans lesquels $R_1$ a la signification ci-dessus, $R_2$ est un hydrogène ou le radical nitro, chlore, cyano, méthoxy ou acétyle, et $R_3$ est un hydrogène, Z est un enchaînement propylène et $R_4$ est le groupe 4-méthyl-2-pyridyle ou le groupe 4-fluorophényle.

5. Composés de 5-alkyl-1-phényl-2-pipérazinoalkyl-pyrazoline-3-one selon la revendication 4, dans lesquels $R_2$, $R_3$, $R_4$ et Z ont les significations données dans la revendication 4, et $R_1$ est un radical alkyle à chaîne droite ou ramifiée ayant de 1 à 4 atomes de carbone ou un radical alkyle cyclique ayant 5 ou 6 atomes de carbone.

6. Composés de 5-alkyl-1-phényl-2-(3-pipérazine-1-ylpropyl)-pyrazoline-3-one selon la revendication 5, dont le noyau 1-phényle est non-substitué ou est substitué en position 4 par un chlore, le groupe nitro ou méthoxy, et dont le groupe 5-alkyle représente le groupe méthyle, éthyle, n-propyle, cyclopentyle ou cyclohexyle.

7. Composés de 5-alkyl-1-phényl-2-(3-pipérazine-1-ylpropyl)-pyrazoline-3-one selon la revendication 5, dont le groupe 5-alkyle $R_1$ est un radical cyclohexyle.

8. Procédé de préparation de composés de 5-alkyl-1-phényl-2-pipérazinoalkylpyrazoline-3-one de formule générale I

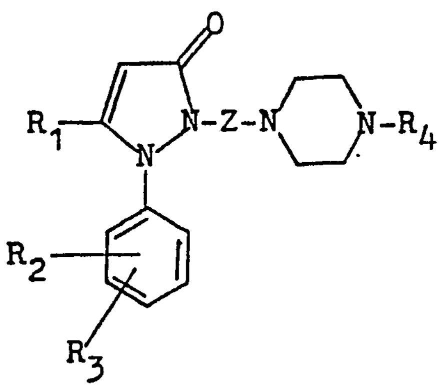

I

dans laquelle $R_1$, $R_2$, $R_3$, $R_4$ et Z ont les significations données dans la revendication 1,
ainsi que leurs sels d'addition avec un acide, caractérisé en ce que

a) on fait réagir des composés de formule II ou III

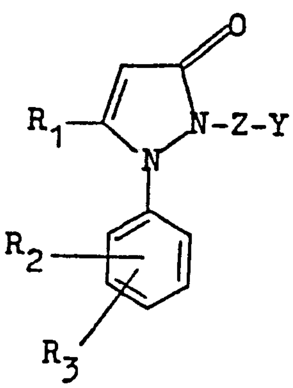

II

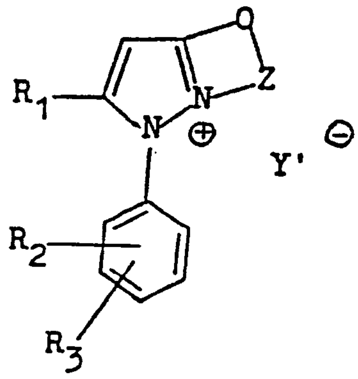

III

dans lesquels $R_1$, $R_2$, $R_3$ et Z ont les significations ci-dessus, Y est un radical éliminable par aminolyse et Y' est un halogène, ou leurs mélanges, avec des composés de formule IV

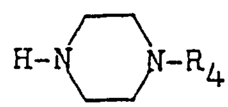

IV

dans laquelle $R_4$ a la signification ci-dessus, ou bien

b) pour préparer des composés de formule générale Ia

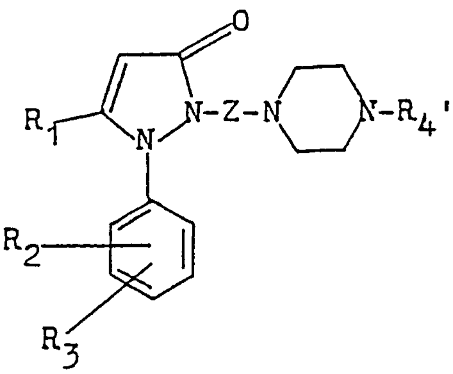

Ia

dans laquelle $R_1$, $R_2$, $R_3$ et Z ont les significations données ci-dessus et $R_4'$ est un groupe 2-pyridyle de formule b'

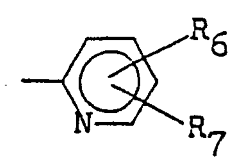

b'

dans laquelle $R_6$ et $R_7$ ont les significations données ci-dessus, on fait réagir des composés de formule V

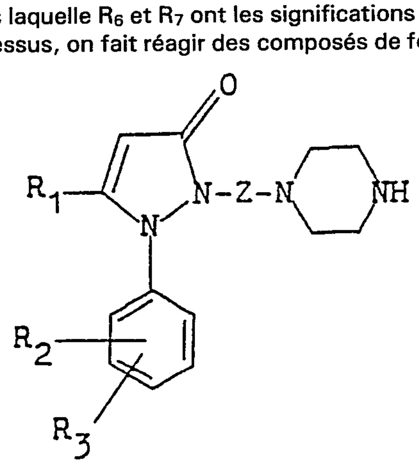

V

dans laquelle $R_1$, $R_2$, $R_3$ et Z ont les significations ci-dessus, avec des composés de formule VI

Hal-$R_4'$ VI

dans laquelle $R_4'$ a la signification ci-dessus et Hal est un halogène, ou bien

c) pour préparer des composés de formule générale Ib

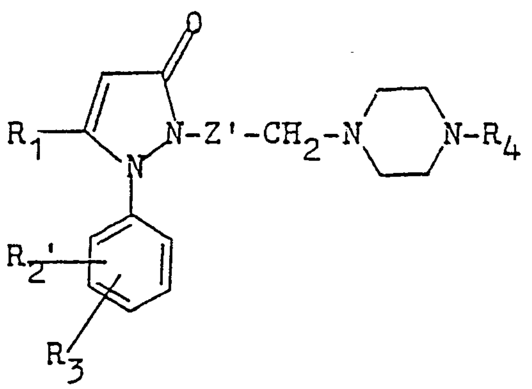

Ib

dans laquelle $R_1$, $R_3$ et $R_4$ ont les significations ci-dessus, $R_2'$ a les significations données pour $R_2$ à l'exception de cyano et de radicaux contenant un groupe CO, et Z' est un enchaînement alkylène ayant 2 ou 3 atomes de carbone,
on réduit des composés de formule VII

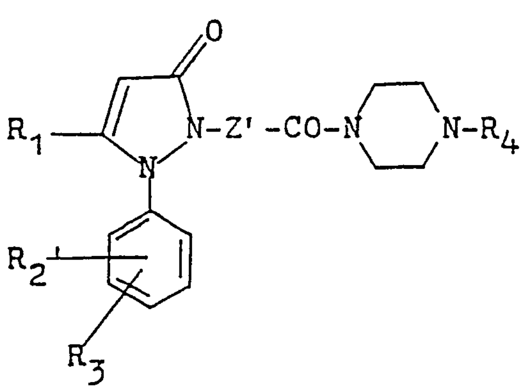

VII

dans laquelle $R_1$, $R_2'$, $R_3$, $R_4$ et Z ont les significations ci-dessus, et
si on le souhaite, dans les composés obtenus de formule I, on libère le groupe hydroxyle des éventuels substituants méthoxy ou le groupe carboxyle des éventuels substituants alcoxycarbonyle, et, éventuellement, on convertit les composés libres de formule I en leurs sels d'addition avec un acide, ou on convertit les sels d'addition avec un acide en composés libres de formule I.

9. Médicament contenant une quantité à effet pharmacologique d'un composé de 5-alkyl-1-phényl-2-pipérazinoalkyl-pyrazoline-3-one selon la revendication 1, et les adjuvants et/ou excipients pharmaceutiques habituels.

10. Composés de formules générales II ou III

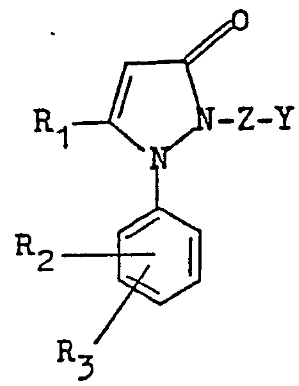

II

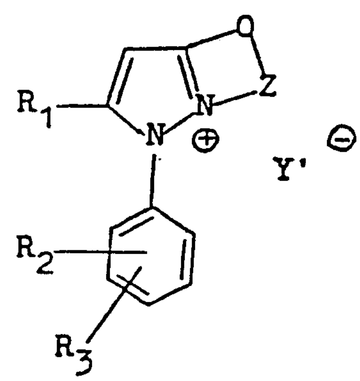

III

dans lesquelles
$R_1$ est un groupe alkyle à chaîne droite, ramifiée ou cyclique, ayant 2 à 6 atomes de carbone, ou encore, si $R_2$ et $R_3$ ne sont pas simultanément des atomes d'hydrogène, représente aussi le radical méthyle,
$R_2$ est un hydrogène, un halogène, le radical nitro ou cyano, ou un radical alkyle ayant de 1 à 4 atomes de carbone, alcoxy ayant de 1 à 4 atomes de carbone ou un groupe $R_5$-CO dans lequel $R_5$ est un groupe alcoxy ayant de 1 à 4 atomes de carbone ou alkyle ayant de 1 à 4 atomes de carbone,
$R_3$ est un hydrogène, un halogène, un alkyle ayant de 1 à 4 atomes de carbone ou un alcoxy ayant 1 à 4 atomes de carbone,
Z est un enchaînement alkylène ayant de 2 à 4 atomes de carbone, et
Y est un radical éliminable par aminolyse et Y' est un halogène.

11. Composé de formule générale V

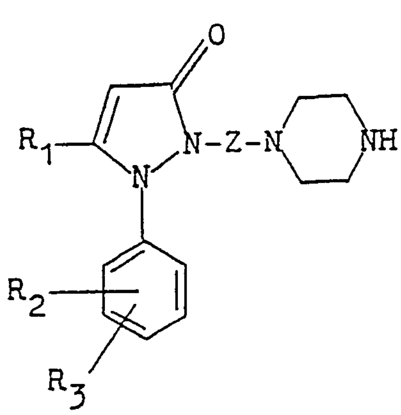

V

dans laquelle

$R_1$ est un groupe alkyle à chaîne droite ou ramifiée, ou cyclique, ayant jusqu'à 6 atomes de carbone,

$R_2$ est un hydrogène, un halogène, le radical nitro, cyano, un radical alkyle ayant de 1 à 4 atomes de carbone, alcoxy ayant de 1 à 4 atomes de carbone, ou un groupe $R_5$-CO dans lequel $R_5$ représente un radical alcoxy ayant de 1 à 4 atomes de carbone, alkyle ayant de 1 à 4 atomes de carbone,

$R_3$ est un hydrogène, un halogène, un alkyle ayant de 1 à 4 atomes de carbone ou un alcoxy ayant de 1 à 4 atomes de carbone,

Z est un enchaînement alkylène ayant de 2 à 4 atomes de carbone.

12. Composés de formule générale VII

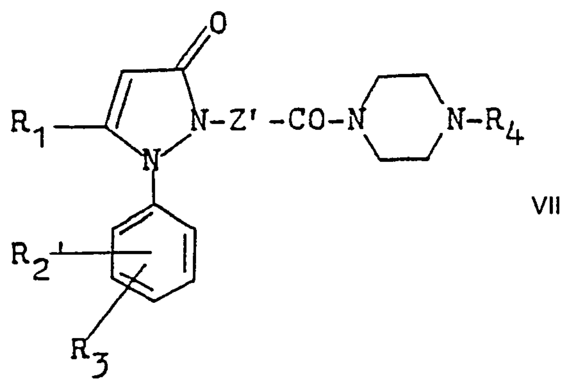

VII

dans laquelle

$R_1$ est un groupe alkyle à chaîne droite ou ramifiée ou cyclique ayant jusqu'à 6 atomes de carbone,

$R_2'$ est un hydrogène, un halogène, le radical nitro, hydroxy, un radical alkyle ayant de 1 à 4 atomes de carbone ou alcoxy ayant de 1 à 4 atomes de carbone,

$R_3$ est un hydrogène, un halogène, un alkyle ayant de 1 à 4 atomes de carbone ou un alkcoxy ayant de 1 à 4 atomes de carbone,

Z' est un enchaînement alkylène ayant 2 ou 3 atomes de carbone,

$R_4$ est un groupe phényle de formule a ou un groupe pyridyle de formule b'

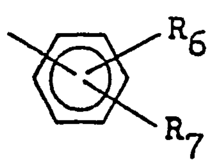

a)

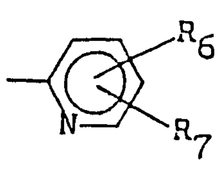

b'

dans laquelle

$R_6$ est un hydrogène, un halogène, un alkyle ayant de 1 à 4 atomes de carbone ou un alcoxy ayant de 1 à 4 atomes de carbone, et

$R_7$ est un hydrogène, un halogène, un alkyle ayant de 1 à 4 atomes de carbone ou un alcoxy ayant de 1 à 4 atomes de carbone,

ainsi que leurs sels d'addition avec un acide.

**Revendications pour l'Etat contractant:** AT

1. Procédé de préparation de composés de 5-alkyl-1-phényl-2-pipérazinoalkylpyrazoline-3-one de formule générale I

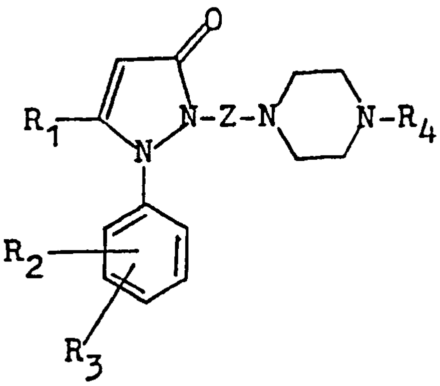

I

dans laquelle

$R_1$ est un groupe alkyle à chaîne droite, ramifiée ou cyclique, ayant jusqu'à 6 atomes de carbone,

$R_2$ est un hydrogène, un halogène, le radical nitro, cyano, hydroxy, un radical alkyle ayant de 1 à 4 atomes de carbone, alcoxy ayant de 1 à 4 atomes de carbone, ou un groupe $R_5$-CO dans lequel $R_5$ représente un radical alcoxy ayant de 1 à 4 atomes de carbone, alkyle ayant 1 à 4 atomes de carbone ou hydroxy,

$R_3$ est un hydrogène, un halogène, un alkyle ayant de 1 à 4 atomes de carbone ou un alcoxy ayant de 1 à 4 atomes de carbone,

Z est un enchaînement alkylène ayant de 2 à 4 atomes de carbone,

$R_4$ est un groupe phényle de formule a ou un groupe pyridyle de formule b

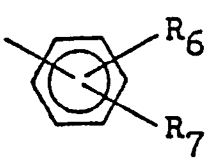

a)

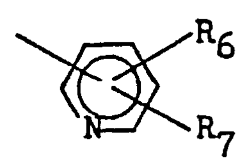

b)

où

$R_6$ est un hydrogène ou halogène, un alkyle ayant de 1 à 4 atomes de carbone ou un alcoxy ayant de 1 à 4 atomes de carbone, et

$R_7$ est un hydrogène, un halogène, un alkyle ayant de 1 à 4 atomes de carbone ou un alcoxy ayant de 1 à 4 atomes de carbone de carbone, ainsi que leurs sels d'addition avec un acide, caractérisé en ce que

a) on fait réagir des composés de formule II ou III

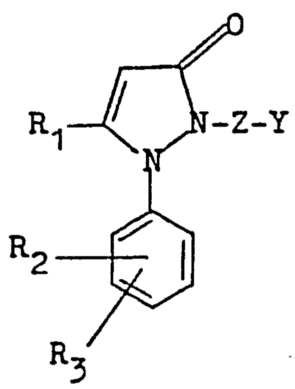

II

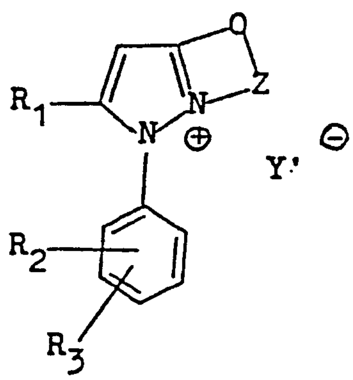

III

dans lesquels $R_1$, $R_2$, $R_3$ et Z ont les significations ci-dessus, Y est un radical éliminable par aminolyse et Y' est un halogène, ou leurs mélanges, avec des composés de formule IV

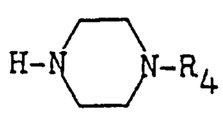

IV

dans laquelle $R_4$ a la signification ci-dessus, ou bien

b) pour préparer des composés de formule générale Ia

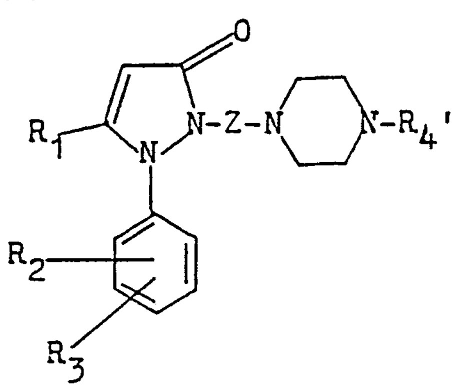

Ia

dans laquelle $R_1$, $R_2$, $R_3$ et Z ont les significations données ci-dessus et $R_4'$ est un groupe 2-pyridyle de formule b'

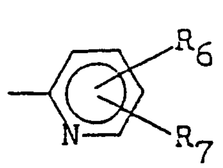

b'

dans laquelle $R_6$ et $R_7$ ont les significations données ci-dessus, on fait réagir des composés de formule V

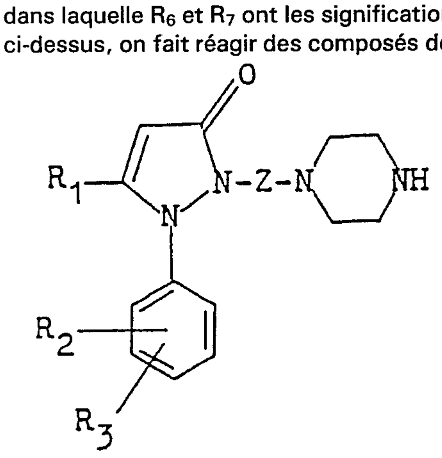

V

dans laquelle $R_1$, $R_2$, $R_3$ et Z ont les significations ci-dessus, avec des composés de formule VI

Hal-$R_4'$ VI

dans laquelle $R_4'$ a la signification ci-dessus et Hal est un halogène, ou bien

c) pour préparer des composés de formule générale Ib

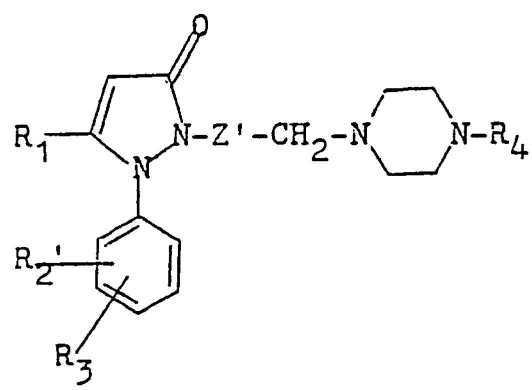

Ib

dans laquelle $R_1$, $R_3$ et $R_4$ ont les significations ci-dessus, $R_2'$ a les significations données pour $R_2$ à l'exception de cyano et de radicaux contenant un groupe CO, et Z' est un enchaînement alkylène ayant 2 ou 3 atomes de carbone,

on réduit des composés de formule VII

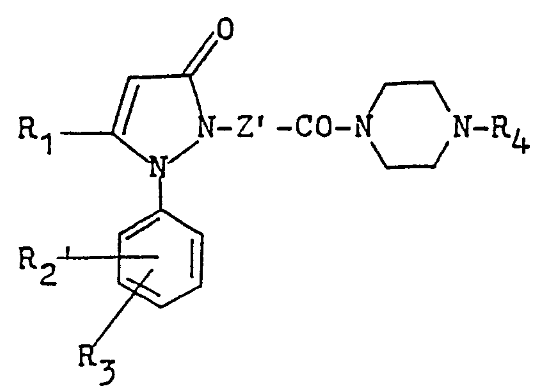

VII

dans laquelle $R_1$, $R_2'$, $R_3$, $R_4$ et Z' ont les significations ci-dessus, et

si on le souhaite, dans les composés obtenus de formule I, on libère le groupe hydroxyle des éventuels

substituants méthoxy ou le groupe carboxyle des éventuels substituants alcoxycarbonyle, et, éventuellement, on convertit les composés libres de formule I en leurs sels d'addition avec un acide, ou on convertit les sels d'addition avec un acide en composés libres de formule I.

2. Procédé selon la revendication 1, caractérisé en ce qu'on prépare en tant que composés de formule I des composés de 5-alkyl-1-phényl-2-pipérazinoalkylpyrazoline-3-one, dans lesquels

$R_1$ et Z ont les significations donnés dans la revendication 1,

$R_2$ est un hydrogène, un halogène, le radical cyano, nitro, hydroxy, ou un radical alkyle ayant de 1 à 4 atomes de carbone, alcoxy ayant de 1 à 4 atomes de carbone, alkylcarbonyle ayant de 2 à 5 atomes de carbone ou alcoxycarbonyle ayant de 2 à 5 atomes de carbone,

$R_3$ est un hydrogène ou un halogène ou un alcoxy ayant de 1 à 4 atomes de carbone,

$R_4$ est un groupe phényle de formule a, où

$R_6$ est un hydrogène ou un halogène, un alcoxy ayant de 1 à 4 atomes de carbone ou un alkyle ayant de 1 à 4 atomes de carbone, et

$R_7$ est un hydrogène ou un halogène, un alkyle ayant de 1 à 4 atomes de carbone ou un alcoxy ayant de 1 à 4 atomes de carbone, ou bien

$R_4$ est un groupe pyridyle de formule b, dans laquelle

$R_6$ est un hydrogène ou un radical alkyle ayant de 1 à 4 atomes de carbone ou un halogène, et

$R_7$ est un hydrogène.

3. Procédé selon la revendication 2, caractérisé en ce qu'on utilise comme composés de formule I des composés de 5-alkyl-1-phényl-2-pipérazinoalkylpyrazoline-3-one, dans lesquels

$R_1$ a la signification ci-dessus,

Z est un enchaînement alkylène ayant 3-4 atomes de carbone,

$R_2$ est un hydrogène, un halogène, le radical cyano, nitro, un radical alcoxy ayant de 1 à 4 atomes de carbone ou alkylcarbonyle ayant de 2 à 5 atomes de carbone,

$R_3$ est un hydrogène, et

$R_4$ est un groupe phényle qui est non-substitué ou est substitué par un halogène, ou bien

$R_4$ est un groupe 2-pyridyle qui est non-substitué ou est substitué par un radical alkyle ayant de 1 à 4 atomes de carbone.

4. Procédé selon la revendication 3, caractérisé en ce qu'on prépare comme composés de formule I des composés de 5-alkyl-1-phényl-2-pipérazinoalkylpyrazoline-3-one, dans lesquels $R_1$ a la signification ci-dessus, $R_2$ est un hydrogène ou un nitro, un chlore, un cyano, un méthoxy ou un acétyle, et $R_3$ est un hydrogène, Z est un enchaînement propylène et $R_4$ est le groupe 4-méthyl-2-pyridyle ou le groupe 4-fluorophényle.

5. Procédé selon la revendication 4, caractérisé en ce qu'on prépare comme composés de formule I des composés de 5-alkyl-1-phényl-2-pipérazinoalkylpyrazoline-3-one, dans lesquels $R_2$, $R_3$, $R_4$ et Z ont les significations donnés dans la revendication 4, et $R_1$ est un radical alkyle à chaîne droite ou ramifiée ayant de 1 à 4 atomes de carbone ou un radical alkyle cyclique ayant 5 ou6 atomes de carbone.

6. Procédé selon la revendication 5, caractérisé en ce qu'on prépare comme composés de formule I des composés de 5-alkyl-1-phényl-2-(3-pipérazine-1-ylpropyl)-pyrazoline-3-one, dont le noyau 1-phényle est non-substitué ou est substitué en position 4 par un chlore, un nitro ou un méthoxy, et dont le groupe 5-alkyle représente le groupe méthyle, éthyle, n-propyle, cyclopentyle ou cyclohexyle.

7. Procédé selon la revendication 6, caractérisé en ce qu'on prépare comme composés de formule I des composés de 5-alkyl-1-phényl-2-(3-pipérazine-1-ylpropyl)-pyrazoline-3-one, dont le groupe 5-alkyle $R_1$ est un radical cyclohexyle.

8. Procédé de préparation de composés de formules générales II ou III,

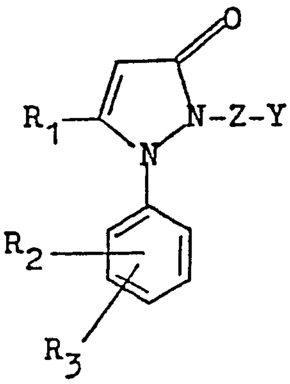

II

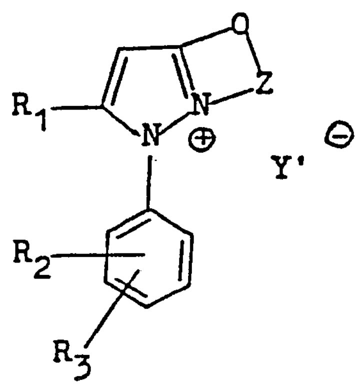

III

dans lesquelles

$R_1$ est un groupe alkyle à chaîne droite ou ramifiée, ou cyclique, ayant 2 à 6 atomes de carbone, ou encore, si $R_2$ et $R_3$ ne sont pas simultanément des atomes d'hydrogène, représente aussi le radical méthyle,

$R_2$ est un hydrogène, un halogène, le radical nitro ou cyano, ou un radical alkyle ayant de 1 à 4 atomes de carbone, alcoxy ayant de 1 à 4 atomes de carbone ou un groupe $R_5$-CO dans lequel $R_5$ est un groupe alcoxy ayant de 1 à 4 atomes de carbone ou alkyle ayant de 1 à 4 atomes de carbone,

$R_3$ est un hydrogène, ou un halogène, un alkyle ayant de 1 à 4 atomes de carbone ou un alcoxy ayant de 1 à 4 atomes de carbone,

Z est un enchaînement alkylène ayant de 2 à 4 atomes de carbone, et

Y est un radical éliminable par aminolyse et Y′ est un halogène,
caractérisé en ce qu'on fait réagir des sels de métaux alcalins, préparés in situ, de composés de formule générale VIII

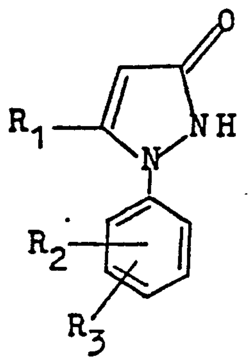

VIII

dans laquelle $R_1$, $R_2$ et $R_3$ ont les significations ci-dessus, avec des composés de formule générale IX

Y-Z-Y′ IX

dans laquelle Z, Y et Y′ ont les significations ci-dessus, et qu'on sépare éventuellement le mélange obtenu d'avec les composés de formule II et III.

9. Procédé pour la préparation de composés de formule générale V

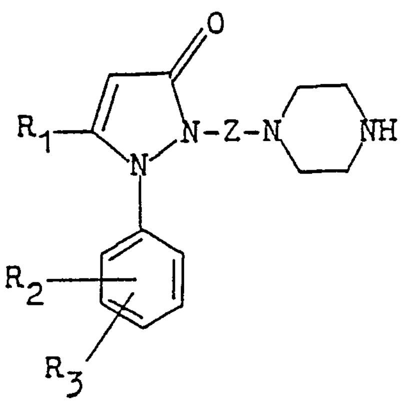

V

dans laquelle

Z est un enchaînement alkylène ayant de 2 à 4 atomes de carbone,

$R_1$ est un groupe alkyle à chaîne droite ou ramifiée, ou cyclique, ayant jusqu'à 6 atomes de carbone,

$R_2$ est un hydrogène, un radical halogène, le radical nitro, cyano, hydroxy, un radical alkyle ayant de 1 à 4 atomes de carbone, alcoxy ayant de 1 à 4 atomes de carbone, ou un groupe $R_5$-CO dans lequel $R_5$ représente un radical alcoxy ayant de 1 à 4 atomes de carbone, alkyle ayant de 1 à 4 atomes de carbone ou hydroxy,

$R_3$ est un hydrogène ou un radical halogène, alkyle ayant de 1 à 4 atomes de carbone ou alcoxy ayant de 1 à 4 atomes de carbone, caractérisé

a) en ce qu'on fait réagir des composés de formules générales II ou III

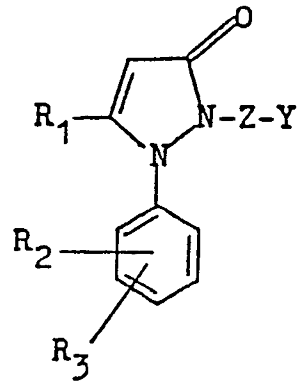

II

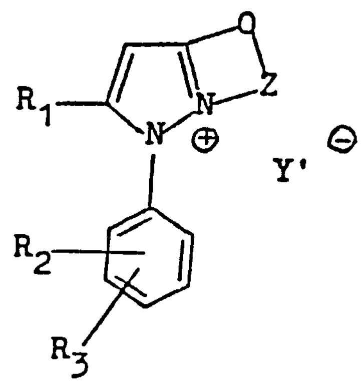

III

dans lesquelles $R_1$, $R_2$, $R_3$ et Z ont les significations ci-dessus, Y est un radical éliminable par aminolyse et Y′ est un halogène, avec un excès de pipérazine, ou bien

b) en ce que, à partir de composés de formule générale X

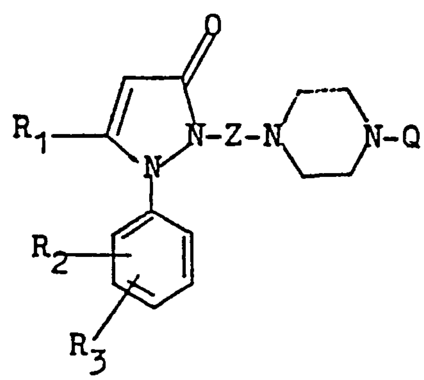

X

dans laquelle $R_1$, $R_2$, $R_3$ et Z ont les significations ci-dessus et Q est un groupe amino-protecteur, on élimine le groupe amino-protecteur.

10. Procédé pour la préparation de composés de formule générale VII

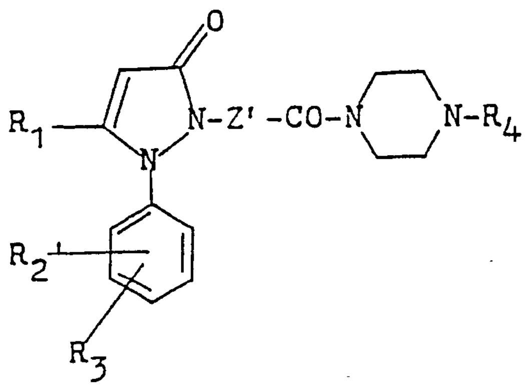

VII

dans laquelle

$R_1$ est un groupe alkyle à chaîne droite ou ramifiée ou cyclique ayant jusqu'à 6 atomes de carbone,

$R_2'$ est un hydrogène, un halogène, le radical nitro, hydroxy, un radical alkyle ayant de 1 à 4 atomes de carbone ou alcoxy ayant de 1 à 4 atomes de carbone,

$R_3$ est un hydrogène, un radical halogéno, alkyle ayant de 1 à 4 atomes de carbone ou alcoxy ayant de 1 à 4 atomes de carbone,

Z' est un enchaînement alkylène ayant 2 ou 3 atomes de carbone,

$R_4$ est un groupe phényle de formule a ou un groupe pyridyle de formule b'

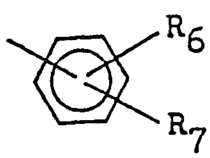

a)

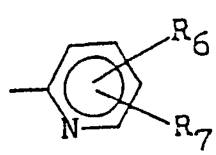

b'

dans laquelle

$R_6$ est un hydrogène, un halogène, un alkyle ayant de 1 à 4 atomes de carbone ou un alcoxy ayant de 1 à 4 atomes de carbone, et

$R_7$ est un hydrogène, un halogène, un alkyle ayant de 1 à 4 atomes de carbone ou un alcoxy ayant de 1 à 4 atomes de carbone,

ainsi que leurs sels d'addition avec un acide, caractérisé en ce qu'on fait réagir des composés de formule générale XIII

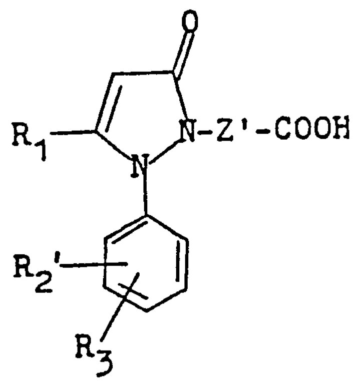

XIII

dans laquelle $R_1$, $R_2$, $R_3$ et Z' ont les significations ci-dessus, avec des composés de formule générale IV

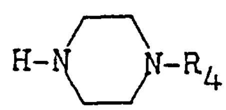

IV

dans laquelle $R_4$ a la signification ci-dessus, et, éventuellement, on convertit les composés libres de formule VII en sels d'addition avec un acide, ou on convertit les sels d'addition avec un acide en composés libres de formule VII.